# EUROPEAN PATENT APPLICATION

(11) **EP 2 100 591 A2**
(43) Date of publication of application: **16.09.2009**
(21) Application number: 09250709.4
(22) Date of filing: 13.03.2009
(51) Int. Cl.: A61K 8/89, A61Q 17/04

(54) **Cosmetic comprising a silicone polymer with a benzotriazol residue**

(30) Priority: 13.03.2008 JP 2008064568; 18.02.2009 JP 2009035018
(71) Applicant: Shin-Etsu Chemical Co., Ltd., Tokyo 100-0004 (JP)
(72) Inventor: Sakuta, Koji, Annaka-shi, Gunma-ken (JP); Tachibana, Kiyomi, Chiyoda-ku, Tokyo (JP); Yamamoto, Aki, Annaka-shi, Gunma-ken (JP)
(74) Representative: Hallybone, Huw George

(57) **Abstract**

A cosmetic, comprising a polymer (A) which comprises a repeating unit represented by the following formula (1), a repeating unit represented by the following formula (2) and a repeating unit represented by the following formula (3), said polymer (A) containing the repeating unit represented by the formula (3) in an amount of from 30 to 90 mass%, based on a total mass of the polymer (A), and being soluble in decamethylcyclopentasiloxane, 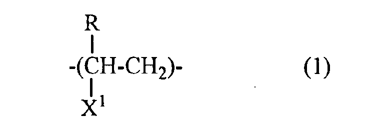 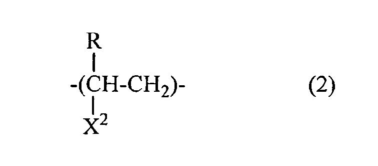 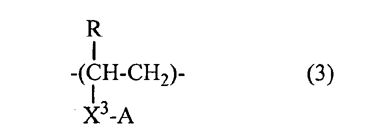 wherein R is a hydrogen atom or a methyl group, X¹ is -COOR¹, a phenyl group or an organic group represented by the following formula (4):

-COO(CₐH₂ₐO)_{b}-R² (4)

X² is an organic group represented by the following formula (5): 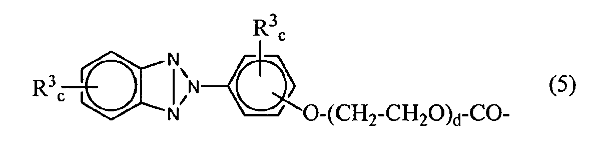 X³ is a C6-10 divalent aryl group or -C(=O)OR⁴- , and A is an organopolysiloxane residue represented by the following formula (6):

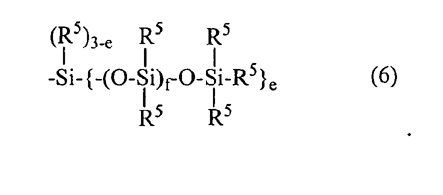

## Description

### FIELD OF THE INVENTION

The present invention relates to a cosmetic comprising a silicone polymer, specifically a polymer which has a silicone residue and a benzotriazol residue to be ultraviolet light protective and durable on the skin.

### BACKGROUND OF THE INVENTION

To prepare ultraviolet light protective cosmetics, hereinafter referred to as "UV protective cosmetics", an UV absorbing agent, UV scattering agent or a combination thereof is incorporated in the cosmetics. Examples of the UV absorbing agent include aminobenzoic acid derivatives, cinnamic acid derivatives, salicylic acid derivative, camphor derivatives, urocanic acid derivatives, benzophenone derivatives, benzotriazol derivatives, dibenzoylmethane derivatives, heterocyclic compound derivatives, and examples of the UV scattering agent include fine particles of titanium dioxide, iron-containing titanium dioxide, zinc oxide, cerium oxide and a composite of these particles.

Meanwhile, methods are known in which a silicone is modified with an UV absorbing agent to improve solubility or dispersibility of the UV absorbing agent in cosmetics, for example, cinnamic acid-modified silicone described in JP3-204887A, styryl ketone -modified silicone described in JP5-43585A, benzophenone -modified silicone described in USP5,270,426, chalcone-modified silicone described in JP5-178865A, benzotriazol-modified silicone described in USP5,089,250, and dibenzoylmethane-modified silicone described in USP5,025,053. These silicone derivatives have improved solubility in cosmetics, but there is still room for improvement in sensory properties to the touch.

USP5,254,542 discloses a silicone resin derivative having a benzotriazolyl group. The silicone derivative has improved compatibility with cosmetic and affinity for the skin, but is tacky to the touch.

### SUMMARY OF THE INVENTION

An object of the present invention is therefore to provide a cosmetic comprising a silicone polymer which has UV absorbing capability, high solubility or dispersibility in unctuous agents used for cosmetics, durability on the skin or hair, and non-tackiness to the touch.

The present invention is a cosmetic, comprising a polymer (A) which comprises a repeating unit represented by the following formula (1), a repeating unit represented by the following formula (2) and a repeating unit represented by the following formula (3), said polymer (A) containing the repeating unit represented by the formula (3) in an amount of from 30 to 90 mass%, based on a total mass of the polymer (A), and being soluble in decamethylcyclopentasiloxane, 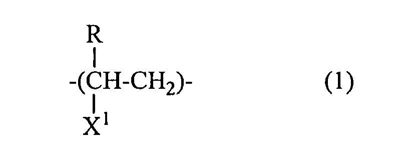 wherein R is a hydrogen atom or a methyl group, X¹ is -COOR¹, wherein R¹ is a hydrogen atom or a C1-10 alkyl group, a phenyl group or an organic group represented by the following formula (4):

-COO(CₐH₂ₐO)_{b}-R² (4)

wherein R² is a hydrogen atom or a C1-30 monovalent hydrocarbon group, which may have an oxygen atom, a is an integer of from 2 to 5, and b is an integer of from 1 to 100; 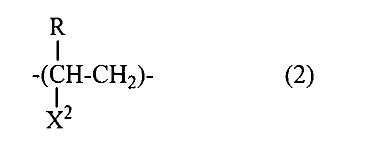 wherein R is as defined above, and X² is an organic group represented by the following formula (5): 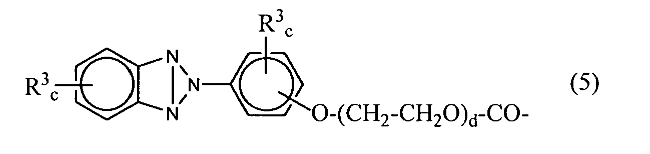 wherein each R³ is, independently, a hydrogen atom, a hydroxyl group, a C1-10 alkyl group or a C1-10 alkoxy group, c is an integer of from 0 to 4, and d is an integer of from 0 to 5; 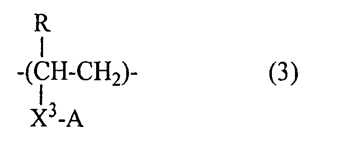 wherein R is as defined above, X³ is a C6-10 divalent aryl group or -C(=O)OR⁴- , wherein R⁴ is a C1-9 alkylene group bonded to A, and A is an organopolysiloxane residue represented by the following formula (6): 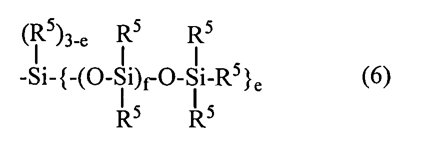 wherein each R⁵ is, independently, a C1-30 alkyl group, aryl group, C1-30 fluorinated alkyl or fluorinated aryl group, e is an integer of from 1 to 3, and f is an integer of from 0 to 300.

In a preferred embodiment, X² in the repeating unit represented by the formula (2) is represented by the following formula (7) or (8):

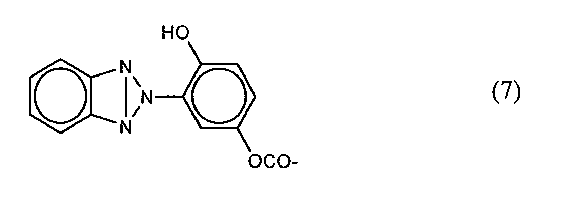

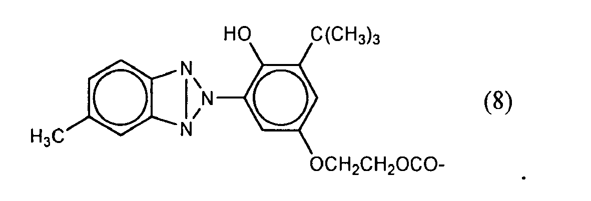

The aforesaid silicone polymer (A) absorbs ultraviolet light and is highly compatible with an unctuous agent. After being applied to the skin, the polymer lasts long on the skin and is not tacky to the touch. Compared with an ordinary UV absorbing agent, the polymer (A) has a larger molecular weight to have a smaller UV absorbance per mass, so that sufficient level of UV protection may not be achieved by the polymer (A) alone. For this reason, it is preferred to use the polymer in combination with a known UV protective component such as a UV absorbing agent or UV scattering agent. In the combinatory use, the polymer (A) improves the UV protective agent's durability on the skin or hair and resistance to water, leading to prolonged UV protective effect. One can make the polymer (A) in the form of liquid or solid with a low or high viscosity as desired by varying ratios of constituent repeating units and/or a degree of polymerization.

### DETAILED DESCRIPTION OF THE PREFERRED EMBODIMENTS

The polymer (A) comprises a repeating unit represented by the formula (1) shown below, hereinafter referred to as the repeating unit (1), a repeating unit represented by the formula (2) shown below, hereinafter referred to as the repeating unit (2), and a repeating unit represented by the following formula (3), hereinafter referred to as the repeating unit (3).

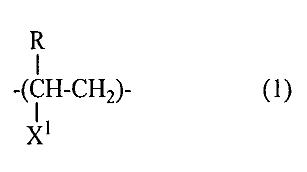

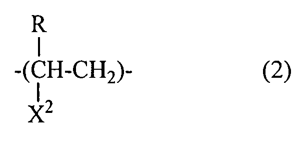

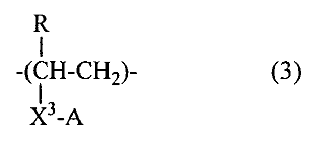

In the repeating units (1), (2), and (3), each R is, independently, a hydrogen atom or a methyl group, and is preferably a methyl group.

In the repeating unit (1), X¹ is -COOR¹, a phenyl group or an organic group represented by the formula (4) shown below, among which -COOR¹ is preferred, wherein R¹ is a hydrogen atom or a C1-10 alkyl group such as methyl, ethyl, n-propyl and isobutyl groups. Preferably, R¹ is a hydrogen atom or a methyl group.

-COO(CₐH₂ₐO)_{b}-R² (4)

In the formula (4), R² is a hydrogen atom or a C1-30, preferably C1-22, monovalent hydrocarbon group which may have an oxygen atom, a is an integer of from 2 to 5, preferably 2 or 3, and b is an integer of from 1 to 100, preferably from 3 to 30.

Examples of the aforesaid monovalent hydrocarbon group include alkyl groups such as methyl, ethyl, n-propyl, n-butyl, 2-ethylhexyl, n-octyl, and n-stearyl groups; alicyclic groups such as cyclohexyl and cyclopentyl groups; butoxyethyl group, benzyl group, tetrahydrofurfuryl group and nonylphenoxy group. Preferably, R² is a hydrogen atom or a methyl group.

Examples of the monomer to derive the repeating unit (1) include alkyl (meth)acrylate such as methyl (meth)acrylate, ethyl (meth)acrylate, butyl (meth)acrylate, cyclohexyl (meth)acrylate, n-octyl (meth)acrylate, 2-ethylhexyl (meth)acrylate, lauryl (meth)acrylate, n-stearyl (meth)acrylate, isostearyl (meth)acrylate, behenyl (meth)acrylate, butoxyethyl (meth)acrylate, benzyl (meth)acrylate, tetrahydrofurfuryl (meth)acrylate, hydroxylethyl (meth)acrylate, polyoxyalkylene mono(meth)acrylate, polyglycerin (meth)acrylate, styrene, polyoxyethylene mono(meth)acrylate, polyoxypropylene mono(meth)acrylate, and poly(oxyethylene/oxypropylene) mono(meth)acrylate.

In the repeating unit (2), X² is an organic group represented by the following formula (5): 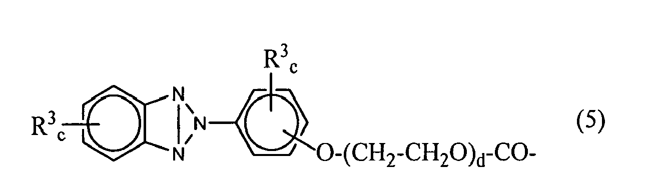 wherein each R³ is, independently, a hydrogen atom, a hydroxyl group, or C1-10, preferably C1-6, alkyl group, or a C1-10, preferably C1-4, alkoxy group. Examples of the alkyl group include methyl, ethyl, n-propyl, n-butyl, t-butyl, and cyclohexyl groups. Examples of the alkoxy group include methoxy, ethoxy, n-propoxy, and n-butoxy groups. Preferably, R³ is a hydrogen atom, hydroxyl group, methyl group, t-butyl group, or methoxy group. In the formula (5), c is an integer of from 0 to 4, preferably from 0 to 2, and d is an integer of from 0 to 5, preferably from 0 to 2.

Particularly preferred X² is an organic group represented by the following formula (7) or (8) each having a benzotriazolyl group.

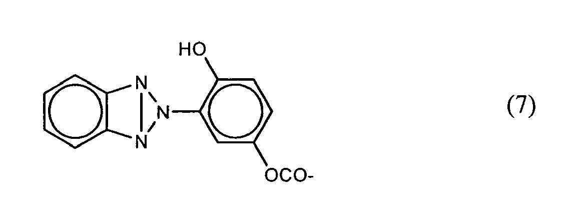

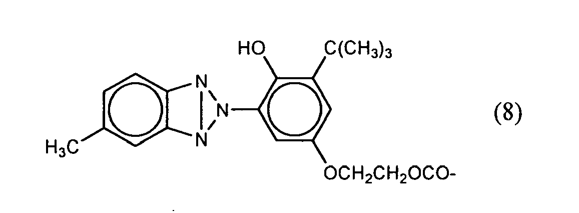

In the repeating unit (3), X³ is a C6-10, preferably C6-8, divalent aryl group or -C(=O)OR⁴-.

Examples of the divalent aryl group include phenylene, tolylene, xylylene and mesitylene groups, among which a phenylene group is preferred.

In the group of the formula, -C(=O)OR⁴-, R⁴ is an alkylene group bonded to A. An example of the alkylene group is represented by the formula, -(CH₂)_{g}-, wherein g is an integer of from 1 to 9, preferably from 2 to 7. Examples of the alkylene group include methylene, ethylene, propylene, butylene groups, among which ethylene and propylene groups are preferred.

In the repeating unit (3), A is an organopolysiloxane residue represented by the following formula (6):

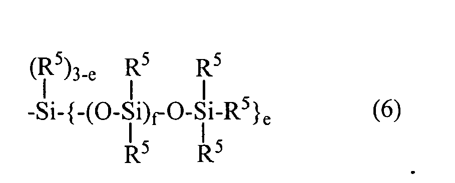

In the formula (6), e is an integer of from 1 to 3, and f is an integer of from 0 to 300. Preferably, e is 1 or 3, and more preferably e is 3 and f is 0, or e is 1 and f is in the range of from 1 to 250, preferably from 5 to 100. Each R⁵ is, independently, C1-30 alkyl, aryl, or fluorinated C1-30 alkyl or aryl groups. Examples of R⁵ include alkyl groups such as methyl, ethyl, propyl, and butyl groups; cycloalkyl groups such as cyclohexyl group; aryl groups such as a phenyl group; and groups obtained by partially or fully fluorinating these groups such as 3,3,3-trifuluoropropyl group. Preferred are methyl, phenyl and fluoropropyl groups. More preferably, at least 90 mole% of R⁵ is methyl group.

The repeating unit (3) constitutes 30 to 95 mass%, preferably 40 to 90 mass%, of the polymer (A), whereby the polymer (A) is soluble in decamethylcyclopentasiloxane. The term "soluble" here means that a mixture of at least 0.1 mass % of the polymer (A) with decamethylcyclopentasiloxane makes a clear solution. A polymer containing the repeating unit (3) in an amount less than the aforesaid lower limit tends to have low solubility in decamethylcyclopentasiloxane. On the other hand, a polymer containing the repeating unit (3) in an amount more than the aforesaid upper limit tends to have inferior durability or long-lasting capability on the skin or hair.

The repeating unit (1) constitutes 0.1 to 70 mass%, preferably 1 to 60mass%, of the polymer (A). A polymer containing the repeating unit (1) in an amount less than the aforesaid lower limit tends to have inferior durability or long-lasting capability on the skin or hair, whereas a polymer containing the repeating unit (1) in an amount more than the aforesaid upper limit tends to have low solubility in decamethylcyclopentasiloxane.

The repeating unit (2) constitutes 0.1 to 50 mass%, preferably 1 to 30mass%, of the polymer (A). A polymer containing the repeating unit (2) in an amount less than the aforesaid lower limit tends to have inferior UV protective capability, whereas a polymer containing the repeating unit (1) in an amount more than the aforesaid upper limit tends to be tacky and have low solubility in decamethylcyclopentasiloxane.

The polymer (A) may comprise repeating units derived from ionic monomers such as anionic, cationic and amphoteric monomers. A content of the ionic monomer is preferably 20 mass% or lower, more preferably 15 mass% or lower relative to a total mass of the polymer (A). A polymer containing more than 20 mass% of the ionic repeating units tends to be tacky and have low solubility in decamethylcyclopentasiloxane.

Examples of the anionic monomer include unsaturated carboxylic acids such as (meth)acrylic acid, maleic acid, maleic acid anhydride, itaconic acid, fumaric acid, and crotonic acid; half esters of unsaturated polybasic acid anhydrides such as succinic acid anhydride or phthalic acid unhydride with (meth)acrylate having hydroxyl group such as hydroxyethyl (meth)acrylate, or hydroxypropyl (meth)acrylate; monomers having sulfonic acid group such as sulfoethyl (meth)acrylate; and monomers having phosphoric acid group such as 2-methacryloyloxyethyl acid phosphate, 2-methacryloyloxypropyl acid phosphate, and 3-chloro-2-acid phosphoxypropyl (meth)acrylate.

Examples of the cationic monomer include compounds derived from (meth)acrylic acid and trialkylamine salt with epihalohydrin, for example, (meth)acryloyloxyhydroxypropyltrimethylammonium chloride and (meth)acryloyloxyhydroxypropyltriethylammonium bromide; amine derivatives of (meth)acrylic acid or (meth)acrylaminde such as dimethylaminoethyl (meth)acrylate, diethylaminoethyl (meth)acrylate, dimethylaminopropyl (meth)acrylate, a compound derived from dimethylaminopropyl (meth)acrylamide and C1-4 dialkylalkanolamine; derivatives of the aforesaid amine derivatives of (meth)acrylic acid or (meth)acrylaminde, for example, obtained by (i)neutralization with hydrochloric acid or lactic acid, (ii) modification with alkyl halide such as methyl chloride, ethyl chloride, methyl bromide, or ethyl iodide,(iii) modification with a halogenated fatty acid ester such as monochloroethyl acetate, or monochloromethyl propionate; and (iv) modification with a dialkyl sulfate such as dimethyl sulfate or diethyl sulfate.

Examples of the amphoteric monomer include amine derivatives of (meth)acrylic acid and (meth)acryl amide such as dimethylaminoethyl (meth)acrylate and dimethylaminopropyl (meth)acrylaminde; monochloroacetic acid salt with triethanolamine, potassium monochloroacetate, and sodium monopropionate modified with halogenated fatty acid or propane sultone. As a nonionic monomer, N-vinyl pyrrolidone can be used.

The polymer (A) can be prepared by polymerizing the monomers to derive the repeating units in the presence of a radical polymerization initiator such as benzoyl peroxide, lauroyl peroxide or azobisisobutyronitrile. The polymerization may be performed in a solvent, for example, aliphatic solvent such as pentane, hexane, decane, dodecane, hexadecane or octadecane; aromatic solvent such as benzene, toluene, or xylene; alcoholic solvent such as methanol, ethanol, propanol, butanol, hexanol, or decanol; halogenated solvent such as chloroform, carbon tetra chloride; or ketone solvent such as acetone, or methyl ethyl ketone. The polymer (A) obtained has a weight average molecular weight determined by GPC with polystyrene standards of from 500 to 500,000, preferably from 3,000 to 100,000.

The polymer (A) is useful for cosmetics, particularly those used for the skin or the hair. The cosmetic include skin care cosmetics such as face milky lotion, cream, face cleansing, pack, oil liquid , massage materials, beautifying lotion, toilet soap, deodorant, hand cream, and lip cream; makeup cosmetics such as face powder, liquid foundation, oil foundation, rouge, eye shadow, mascara, eyeliner, eye liner and lipstick; and hairdressing cosmetics such as shampoo, rinse, treatment, and setting agent; antiperspirant and UV protective cosmetics such as s UV protective milky lotion and UV protective cream. A content of the polymer in a cosmetic can be varied according to the form of the cosmetic and ranges from 0.5 to 99.0 mass %, preferably from 1.0 to 50 mass% relative to a total weight of the cosmetic.

In addition to the polymer (A), the present cosmetic may contain various components of conventional cosmetic such as an UV-protective agent (B) and unctuous agent (C). Particularly preferred is the UV-protective agent (B). Each component is explained below.

Examples of the UV-protective agent (B) include UV absorbents and UV scattering agents. Examples of the UV absorbents include UV absorbents of benzoic acid type such as p-aminobenzoic acid; those of anthranilic acid type such as methyl anthranilate; those of salicylic acid type such as methyl salicylate; those of succinic acid type such as octyl p-methoxysuccinate; those of benzophenone type, such as 2,4-dihydroxybenzophenone; those of urocanic acid type such as ethyl urocanate; and those of dibenzoylmethane type such as 4-t-butyl-4'-methoxydibenzoylmethane. The aforesaid silicone derivatives having UV absorbing substituent group can be used.

Examples of the UV scattering agents include fine powder of titanium oxide, fine powder of iron-containing titanium oxide, fine powder of zinc oxide, fine powder of cerium oxide, and a mixture of these powders. Among these, succinic acid type UV absorbents, dibenzoylmethane type UV absorbents, fine powder of titanium oxide and fine powder of zinc oxide are preferred.

As the unctuous agent (C), any unctuous agents can be used, whether it is solid, semi-solid or liquid, which are commonly used in cosmetics.

Examples of liquid unctuous agents include silicone oils, hydrocarbon oils, ester oils, natural plant or animal oils, semi-synthetic oils and fluorinated oils.

Examples of silicone oils include linear or branched organopolysiloxanes having a low viscosity to a high viscosity such as dimethylpolysiloxane, caprylylmethicone, phenyltrimethycone, methylphenylpolysiloxane, methylhexylpolysiloxane, methylhydrogenpolysiloxane and a copolymer of dimethylsiloxane and methylphenylsiloxane; cyclic siloxanes such as octamethylcyclotetrasiloxane, decamethylcyclopentasiloxane, dodecamethylcyclohexasiloxane, tetramethyl-tetrahydrogencyclotetrasiloxane; tetramethyltetraphenylcyclotetrasiloxane; branched ortanopolysiloxane such as tristrimethylsiloxymethylsilane, and tetraquistrimethylsiloxysilane; amino-modified organopolysiloxane; silicone rubber such as gummy dimethylpolysiloxanes having high polymerization degrees, gummy amino-modified organopolysiloxane, and gummy dimethylsiloxane/methylphenylsiloxane copolymer; cyclosiloxane solutions of silicone gum or rubber, trimethylsiloxysilicate, cyclosiloxane solutions of trimethylsiloxysilicate, higher alkoxy-modified silicones such as stearoxysilicone, higher fatty acid-modified silicones, alkyl-modified silicones, long alkyl chain-modified organopolysiloxane, fluorine-modified silicones, and solutions of silicone resins in a cyclic siloxane.

Examples of the hydrocarbon oils include ozokerite, α-olefin oligomer, light isoparaffin, isododecane, light liquid isoparaffin, squalane, synthetic squalane, plant-origin sualane, squalene, ceresin, paraffin, paraffin wax, polyethylene wax, polyethylene/polypropylene wax, ethylene/propylene/styrene copolymer, butylene/propylene/styrene copolymer, liquid paraffin, liquid isoparaffin, pristane, polyisobutylene, hydrogenated polyisobutene, microcrystalline wax, and Vaseline.

Examples of higher fatty acids include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, and 12-hydroxystearic acid.

Examples of the higher alcohols include lauryl alcohol, myristyl alcohol, palmityl alcohol, stearyl alcohol, behenyl alcohol, hexadecyl alcohol, oleyl alcohol, isostearyl alcohol, hexyldodecanol, octyl dodecanol, cetostearyl alcohol, 2-decyltetradecinol, cholesterol, phytosterol, POE cholesterol ether, monostearyl glycerin ether (batyl alcohol), and monooleyl glyceryl ether (cerakyl alcohol).

Examples of ester oils include diisobutyl adipate, 2-hexyldecyl adipate, di-2-heptylundecyl adipate, N-alkyl glycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, isononyl isononanate, neopentyl glycol dicaprirate, triethyl citrate, 2-ethylhexyl succinate, amyl acetate, ethyl acetate, butyl acetate, isocetyl stearate, butyl stearate, diisopropyl sebacinate, di-2-ethylhexyl sebacinate, cetyl lactate, myristyl lactate, isopropyl palmitate, 2-ethylhexyl palmitate, 2-hexyldecyl palmitate, 2-heptylundecyl palmitate, cholesteryl 12-hydroxystearate, dipentaerythritol fatty acid esters, isopropyl myristate, octyldodecyl myristate, 2-hexyldecyl myristate, myristyl myristate, hexyldecyl dimethyloctanoate, ethyl laurate, hexyl laurate, 2-octyldodecyl N-lauroyl-L-glutamate, and diisostearyl. Examples of glyceride oils include acetoglyceryl, glycerol triisooctanoate, glyceryl triisostearate, glyceryl triisopalmitate, glyceryl monostearate, glyceryl di-2-heptylundecanoate, glyceryl trimyristate, and diglyceryl myristyl isostearate.

Examples of natural plant or animal oils and semi-synthetic oils include avocado oil, linseed oil, almond oil, Ibota wax, perilla oil, olive oil, cacao butter, kapok wax, kaya oil, carnauba wax, Glycyrrhiza oil, candelilla wax, beef tallow, neat's-foot oil, beef bone fat, hydrogenated beef tallow, apricot kernel oil, spermaceti wax, hydrogenated oil, wheat germ oil, sesame oil, rice germ oil, rice bran oil, sugar cane wax, sasanqua oil, safflower oil, shear butter, Chinese tung oil, cinnamon oil, jojoba wax, shellac wax, turtle oil, soybean oil, tea seed oil, camellia oil, evening primrose oil, corn oil, lard, rapeseed oil, Japanese tung oil, rice bran oil, germ oil, horse fat, persic oil, palm oil, palm kernel oil, castor oil, hydrogenated castor oil, castor oil fatty acid methylester, sunflower oil, grape oil, bayberry wax, jojoba oil, macadamia nut oil, beeswax, mink oil, cottonseed oil, cotton wax, Japanese wax, Japanese wax kernel oil, montan wax, coconut oil, hydrogenated coconut oil, tri-coconut oil fatty acid glyceride, mutton tallow, peanut oil, lanolin, liquid lanolin, hydrogenated lanolin, lanolin alcohol, hard lanolin, lanolin acetate, isopropyl lanolate, hexyl laurate, POE lanolin alcohol ether, POE lanolin alcohol acetate, polyethylene glycol lanolate, POE hydrogenated lanolin alcohol ether, and egg yolk oil, wherein POE represents polyoxyethylene.

Examples of fluorine-containing oil include perfluoropolyether, perfluorodecalin, and perfluorooctane.

A content of the unctuous agents(C) in the cosmetic ranges from 1 to 98 mass%, preferably from 1 to 50 mass % relative to a total mass of the cosmetic.

The present cosmetic may comprise (D) water according to an intended use of the cosmetic. A content of water in the cosmetic ranges preferably from 0 to 95 mass% relative to a total mass of the cosmetic.

The cosmetic of the present invention may contain a compound having an alcoholic hydroxyl group (E) except aforesaid higher alcohol. Examples of the compound having an alcoholic hydroxyl group (E) include lower alcohols such as ethanol, propanol, and isopropanol; sugar alcohols such as sorbitol, maltose, and maltitol; sterols such as cholesterol, sitosterol, phytosterol, and lanosterol; polyalcohols such as butylene glycol, propylene glycol, dibutylene glycol, and pentylene glycol. Usually, water soluble monohydric alcohol or polyhydric alcohol is used. A content of the compound having an alcoholic hydroxyl group in the cosmetic ranges preferably ranges from 0 to 98 mass% relative to a total mass of the cosmetic.

The present cosmetic may contain a water-soluble or water-swellable polymer (F). Examples of the water-soluble or water-swellable polymer include gum Arabic, tragacanth gum, arabinogalactan, locust bean gum (carob gum), guar gum, karaya gum, carrageenan, pectin, agar-agar, quince seed (i.e., marmelo), starch from rice, corn, potato or wheat, algae colloid, and trant gum; bacteria-derived polymers such as xanthan gum, dextran, succinoglucan, and pullulan; animal-derived polymers such as collagen, casein, albumin, and gelatin; starch-derived polymers such as carboxymethyl starch and methylhydroxypropyl starch; cellulose polymers such as methyl cellulose, ethyl cellulose, methylhydroxypropyl cellulose, carboxymethyl cellulose, hydroxymethyl cellulose, hydroxypropyl cellulose, nitrocellulose, sodium cellulose sulfate, sodium carboxymethyl cellulose, crystalline cellulose, and cellulose powder; alginic acid-derived polymers such as sodium alginate and propylene glycol alginate; vinyl polymers such as polyvinyl methylether, polyvinylpyrrolidone, and carboxyvinyl polymer; polyoxyethylene polymers such as polyethylene glycol; polyoxyethylene/polyoxypropylene copolymers; acrylic polymers such as sodium polyacrylate, polyethyl acrylate, and polyacrylamide; polyethyleneimine; cationic polymers; and inorganic water-soluble polymer such as, bentonite, aluminum magnesium silicate, montmorillonite, videlite, nontronite,saponite, hectorite, and silicic anhydride. Film forming polymers such as polyvinyl alcohol and polyvinylpyrrollidone are also included. An amount of the water-soluble or water-swellable polymer (F) in the cosmetic ranges preferably from 0 to 25 mass% relative to a total mass of the cosmetic.

The present cosmetic may contain a powder (G). Any powder which is commonly used in cosmetics may be used, regardless of the shape such as spherical, rod-like, acicular, or flake, particle size such as fume grade, fine grade or pigment grade, and particle structure such as porous and non-porous. Examples of the powder include inorganic powder, organic powder, surface active metal salt powder, colored pigments, pearl pigments, metallic powder, natural colorants, and coloring agent such as dyes.

Examples of the inorganic powder include inorganic powder include titanium dioxide, zirconium oxide, zinc oxide, cerium oxide, magnesium oxide, barium sulfate, calcium sulfate, magnesium sulfate, calcium carbonate, magnesium carbonate, talc, mica, kaolin, sericite, white mica, synthetic mica, phlogopite, lepidolite, biotite, lithia mica, silicic acid, silicic anhydride, aluminum silicate, magnesium silicate, aluminum magnesium silicate, calcium silicate, barium silicate, strontium silicate, metal salts of tungstenic acid, hydroxyapatite, vermiculite, higilite, bentonite, montmorillonite, hectolitre, zeolite, ceramics powder, calcium secondary phosphate, alumina, aluminum hydroxide, boron nitride, silica and silylated silica.

Examples of the organic powder include polyamide powder, polyester powder, polyethylene powder, polypropylene powder, polystyrene powder, polyurethane powder, benzoguanamine powder, polymethylbenzoguanamine powder, tetrafluoroethylene powder, polymethylmethacrylate powder, cellulose powder, silk powder, powder of nylon such as Nylon 12 and Nylon 6, crosslinked dimethylpolysiloxane fine powder, crosslinked spherical polymethylsilsesquioxane fine powder, crosslinked spherical organopolysiloxane rubber or elastomer coated with polymethylsilsesquioxane particulates, hydrophobic silica, powder of styrene / acrylic acid copolymer, divinylbenzene /styrene copolymer, vinyl resin, urea resin, phenol resin, fluororesin, silicone resin, acrylic resin, melamine resin, epoxy resin, polycarbonate resin, microcrystalline fiber, starch powder, and lauroyl lysine.

Examples of the powder of metal salt surfactant, i.e. metal soap, include zinc undecylenate, aluminum isostearate, zinc stearate, aluminum stearate, calcium stearate, magnesium stearate, zinc myristate, magnesium myristate, zinc cetyl phosphate, calcium cetyl phosphate, zinc sodium cetyl phosphate, zinc palmitate, aluminum palmitate and zinc laurate.

Examples of the colored pigments include inorganic red pigments such as iron oxide, iron hydroxide, and iron titanate, inorganic brown pigments such as γ- iron oxide, inorganic yellow pigments such as iron oxide yellow and loess, inorganic black pigments such as iron oxide black and carbon black, inorganic violet pigments such as manganese violet and cobalt violet, inorganic green pigments such as chromium hydroxide, chromium oxide, cobalt oxide, and cobalt titanate, inorganic blue pigments such as Prussian blue and ultramarine blue, lakes of tar pigments, lakes of natural dyes, and composite of these powder with a synthetic resin powder.

Examples of the pearl pigments include titanium dioxide-coated mica, bismuth oxychloride, titanium dioxide-coated bismuth oxychloride, titanium dioxide-coated talc, fish scales, and titanium dioxide-coated colored mica; metallic powder pigments such as aluminum powder, copper powder and stainless steel powder.

Examples of the tar pigments include Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, and Orange No. 207; and natural pigments such as carminic acid, laccaic acid, carthamin, brazilin, and crocin.

In the present invention, it is preferred that at least part of the powder is a spherical fine powder of crosslinked dimethylpolysiloxane , a spherical fine powder of crosslinked polymethylsilsesquioxane, or a fine powder of crosslinked spherical polysiloxane rubber or elastomer powder coated with polymethylsilsesquioxane particulates. A powder or colorant having fluorinated groups may be used.

The powder may be in the form of composite or may be treated with silicone oil, fluorine compound, or surfactant to the extent not to adversely affect the present cosmetics. For example, the powder may or may not be treated beforehand with fluorine compound, silicone resin, silane coupling agent, titanium coupling agent, unctuous agent, N-acyl lysine, polyacrylic acid, metal surfactant, amino acid, inorganic compound; treatment of pendants; plasma treatment, or mechanochemical treatment. Two or more of the treatment may be employed. The powder can be incorporated in the present cosmetic in an amount not more than 99 mass % relative to a total mass of the cosmetic. Particularly in the powder cosmetic, the powder is preferably incorporated in an amount of from 80 to 99 mass% of the cosmetic.

The present cosmetic may contain one or more of a surfactant (H), depending on intended use of cosmetic. Surfactant commonly used in cosmetics may be used, for example, anionic, cationic, nonionic or amphoteric surfactant.

Examples of the anionic surfactant include fatty acid soaps, such as sodium stearate and triethanolamine palmitate, alkylether carboxylic acids and salts thereof, salts of condensates of amino acids with fatty acids, alkyl sulfonate salts, alkenesulfonates, sulfonates of fatty acid esters, fatty acid amide sulfonates, sulfonate salts of the formalin condensates, salts of alkyl sulfates, salts of secondary higher alcohol sulfates, salts of alkyl/allyl ether sulfates, salts of fatty acid ester sulfates, salts of fatty acid alkylolamide sulfates, and salts of Turkey Red oil sulfate, alkyl phosphate salts, ether phosphate salts, alkylallylether phosphate salts, amide phosphate salts, and N-acylamino surfactants.

Examples of the cationic surfactants include amine salts such as alkylamine salts, amine salts of polyamine and amino alcohol fatty acid derivatives, alkyl quaternary ammonium salts, aromatic quaternary ammonium salts, pyridinium salts and imidazolium salts.

Examples of the nonionic surfactants include sorbitan fatty acid esters, glycerin fatty acid esters, polyglycerin fatty acid esters, propylene glycol fatty acid esters, polyethylene glycol fatty acid esters, sucrose fatty acid esters, polyoxyethylene alkyl ethers, polyoxypropylene alkyl ethers, polyoxyethylene alkyl phenyl ether, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene phytostanol ether, polyoxyethylene phytosterol ether, polyoxyethylene cholestanol ether, polyoxyethylene cholesteryl ether, linear or branched-polyoxyalkylene- modified organopolysiloxane, linear or branched polyoxyalkylene/alkyl co-modified organopolysiloxane, linear or branched polyglycerin-modified organopolysiloxane, linear or branched polyglycerin/alkyl co-modified organopolysiloxane, alkanolamide, sugar ethers, and sugar amides.

Examples of the amphoteric surfactant include betaine, aminocarboxylates, imidazoline derivatives, and amide amine type.

Among the surfactants, preferred are a linear or branched organopolysiloxane having a polyoxyethylene group, a linear or branched organopolysiloxane having a polyglycerin moiety, co-modified organopolysiloxane having an alkyl group in addition to the polyoxyethylene or polyglycerin moiety, and surfactants having HLB of from 2 to 10. A content of the surfactant preferably ranges from 0.1 to 20 mass%, particularly from 0.2 to 10 mass% relative to a total mass of the cosmetic.

The cosmetics of the present invention may contain a composition consisting of a crosslinked organopolysiloxane having no hydrophilic group and liquid unctuous agent (I). The crosslinked organopolysiloxane can be obtained by reacting an alkylhydrogenpolysiloxane with a crosslinking agent having reactive vinyl group. Examples of the alkylhydrogenpolysiloxane include linear and partly branched methylhydrogenpolysiloxanes and methylhydrogenpolysiloxane having C₆₋₂₀ alkyl chains grafts. The alkylhydrogenpolysiloxane should have at least two, on average, hydrogen atoms per molecule. Examples of the crosslinking agent include methylvinylpolysiloxane and alkenyldiene which have at least two vinyl groups per molecule. Examples of the crosslinked organopolysiloxane are those described in JP No.1925781B, JP No. 1932769, and WO 03-24413. The crosslinked organopolysiloxane is swollen with the liquid unctuous agent such as a silicone oil having a viscosity of from 0.65mm²/sec (25°C) to 100.0mm²/sec(25°C), liquid paraffin, squalane, hydrocarbon oil such as isododecane, glyceride oil such as trioctanoyne, and ester oils. The crosslinked organopolysiloxane swollen with oil is contained preferably in the cosmetic in an amount of from 0.1 to 50 mass%, more preferably from 1 to 30 mass%, and still more preferably from 1 to 30 mass% relative to a total mass of the cosmetics.

The cosmetic of the present invention may contain a composition consisting of a liquid unctuous agent and a crosslinked organopolysiloxane having a hydrophilic group (J). Preferred hydrophilic groups are polyoxyether moiety and a polyglycerin moiety. Such crosslinked organopolysiloxane can be obtained by reacting an alkylhydrogenpolysiloxane with a crosslinking agent having reactive vinyl groups. Examples of the alkylhydrogenpolysiloxane include methylhydrogenpolysiloxanes grafted with polyoxyethylene chains and methylhydrogenpolysiloxane grafted with polyglycerin chains. The alkylhydrogenpolysiloxane should have at least two, on average, hydrogen atoms per molecule. The crosslinked organopolysiloxane is swollen with liquid unctuous agent such as silicone oil having a viscosity of from 0.65mm²/sec (25°C) to 100.0mm²/sec(25°C), liquid paraffin, squalane, hydrocarbon oil such as isododecane, glyceride oil such as trioctanoyne, and ester oils.

Examples of the crosslinking agent include methylvinylpolysiloxane, α,ω-alkenyldiene, glycerin triallyl ether, polyoxyalkynylated glycerin trially ether, trimethylolpropane trially ether, polyoxyalkynylated trimethylolpropane trially ether. The crosslinked organopolysiloxane preferably has at least one selected from the group consisting of polyoxyalkylene group, polyglycerin residue, alkyl group, alkenyl group, aryl group, and fluoroalkyl group. Preferred examples of the composition (J) are those described in JP No.2631772B, JP No.9-136813A, JP No.2001-342255A, and WO 03/20828.

The present cosmetic may contain a silicone resin (K) selected from the group consisting of silicone network compounds containing SiO₂ units and/or RSiO_{1.5}, wherein R is an alkyl group, and a linear acrylic silicone graft- or block-copolymer. The linear acrylic silicone graft- or block-copolymer may contain at least one selected from the group consisting of pyrrolidonyl group, long-chain alkyl group, polyoxyalkylene group, fluoroalkyl group, and anionic moiety such as carboxyl group.

Preferred silicone network compounds include those represented by MQ, MDQ, MT, MDT, MDTQ, wherein M stands for R₃ SiO_{0.5} unit, D for R₂ SiO unit, T for RSiO_{1.5} unit, and Q for SiO₂ unit, wherein R is a hydrogen atom or an organic group. The silicone network compounds may have at least one selected from the group consisting of pyrrolidonyl group, long-chain alkyl group, polyoxyalkylene group, fluoroalkyl group, and amino group.

The silicone resin such as the acrylic silicone resin or the silicone network compound is incorporated in the cosmetic in the form of resinous solid or in the form of a solution dissolved in silicone oil having low-viscosity or other kind of solvent. The silicone resin is contained in the cosmetic in an amount of, as solid, preferably from 0.1 to 20 mass%, more preferably from 1 to 10 mass% relative to a total mass of the cosmetic.

In the cosmetic of the present invention, other components that are commonly used in cosmetics can be incorporated in an amount not to adversely affect the cosmetic. Examples of the components include oil-soluble gelling agents, clay minerals modified with organic compounds, resins, antiperspirants, ultraviolet absorbents, ultraviolet absorbing and scattering agents, moisture retention agents, antiseptics, anti-microbial agents, perfumes, salts, antioxidants, pH regulators, a chelating agents, refreshing agents, an anti-inflammatory agent, skin beautifying components, such as skin whitener, cell activator, rough dry skin improver, blood circulation promoter, skin astringent and anti-seborrheic agent, vitamins, amino acids, nucleic acids, hormones, clathrate compounds, and hair setting agents.

Examples of the oil-soluble gelling agent include metal soaps, such as aluminum stearate, magnesium stearate and zinc myristate; amino acid derivatives, such as N-lauroyl-L-glutamic acid and α, γ -di-n-butylamine; dextrin fatty acid esters, such as dextrin palmitic acid ester, dextrin stearic acid ester and dextrin 2-ethylhexaminic acid palmitic acid ester; inulin fatty acid esters such as fructooligostearate; sucrose fatty acid esters, such as sucrose palmitic acid ester and sucrose stearic acid ester; benzylidene derivatives of sorbitol, such as monobenzylidene sorbitol and dibenzylidene sorbitol; and clay minerals modified with organic compounds, such as dimethylbenzyldodecyl ammonium montmorillonite clay and dimethyldioctadecyl ammonium montmorillonite clay.

Examples of the antiperspirant include aluminum chlorohydrate, aluminum chloride, aluminum sesquichlorohydrate, zirconium hydoxychloride, aluminum zirconium hydroxychloride, and aluminum zirconium glycine complex.

Examples of moisture retention agents include glycerin, sorbitol, propylene glycol, dipropylene glycol, 1,3-butylene glycol, pentylene glycol, glucose, xylitol, maltitol, polyethylene glycol, hyaluronic acid, chondroitin sulfuric acid, pyrrolidone carboxylate, polyoxyethylene glycoside, and polyoxypropylene methylglycoside.

Examples of the antiseptics include alkyl paraoxybenzoates, benzoic acid, sodium benzoate, sorbic acid, potassium sorbate, and phenoxyethanol may be used. For the antibacterial agents, benzoic acid, salicylic acid, carbolic acid, sorbic acid, paraoxybenzoic acid alkyl esters, parachloromethacresol, hexachlorophene, benzalkonium chloride, chlorohexydine chloride, trichlorocarbanilide and phenoxyethanol.

Examples of the salts include inorganic salts, organic acid salts, salts of amine and salts of amino acids. Examples of the inorganic salts include sodium, potassium, magnesium, calcium, aluminum, zirconium, or zinc salt of inorganic acid such as hydrochloric acid, sulfuric acid, carbonate acid, and nitric acid. Examples of the salts of organic acid include salts of organic acid such as acetic acid, dehydroacetic acid, citric acid, malic acid, succinic acid, ascorbic acid, and stearic acid. Examples of the salts of amine or amino acid include salt of triethanol amine and salt of glutamic acid. Other examples are salt of hyaluronic acid, chondroitin sulfate, aluminum/zirconium/glycine chelate, and salts produced by acid-alkaline neutralization reaction in the cosmetic.

Examples of the antioxidants include tocopherol, butylhydroxyanisole, dibutylhydroxytoluene and phytic acid; examples of the pH regulators include lactic acid, citric acid, glycolic acid, succinic acid, tartaric acid, dl-malic acid, potassium carbonate, sodium hydrogen carbonate and ammonium hydrogen carbonate; examples of the chelating agents include alanine, sodium ethylenediamine tetraacetate, sodium polyphosphate, sodium metaphosphate and phosphoric acid; examples of the refrigerants include L-menthol and camphor; and examples of the anti-inflammatory agents include allantoin, glycyrrhizin and salts thereof, glycyrrhetinic acid and stearyl glycyrrhetinate, tranexamic acid and azulene.

Examples of the skin-beautifying components include whitening agents, such as placenta extract, arbutin, glutathione and Yukinoshita extract; cell activators, such as royal jelly, photosensitizers, cholesterol derivatives and calf blood extract; rough and dry skin improvers; blood circulation improvers, such as nonylic acid vanillyl amide, benzyl nicotinate, beta -butoxyethyl nicotinate, capsaicin, zingerone, cantharis tincture, ichtammol, caffeine, tannic acid, alpha-borneol, tocopheryl nicotinate, inositol hexanicotinate, cyclandelate, cinnarizine, tolazoline, acetyl choline, verapamil, cepharanthin and gamma -oryzanol; skin astringents, such as zinc oxide and tannic acid; and anti-seborrheic agents, such as sulfur and thianthol.

Examples of the vitamins include vitamin A, such as vitamin A oil, retinol, retinyl acetate and retinyl palmitate; vitamin B, including vitamin B₂ such as riboflavin, riboflavin butyrate and flavin adenine nucleotide, vitamin B₆ such as pyridoxine hydrochloride, pyridoxine dioctanoate and pyridoxine tripalmitate, vitamin B₁₂ and its derivatives, and vitamin B15 and its derivatives; vitamin C, such as L-ascorbic acid, L-ascorbic acid dipalmitic ester, sodium (L-ascorbic acid)-2-sulfate and dipotassium L-ascorbic acid diphosphate; vitamin D, such as ergocalciferol and cholecarciferol; vitamin E, such as alpha -tocopherol, beta -tocopherol, gamma -tocopherol, dl-alpha -tocopheryl acetate, dl- alpha -tocopheryl nicotinate and dl- alpha -tocopheryl succinate; vitamin H; vitamin P; nicotinic acids, such as nicotinic acid, benzyl nicotinate and nicotinic acid amide; pantothenic acids, such as calcium pantothenate, D-pantothenyl alcohol, pantothenyl ethyl ether and acetylpantothenyl ethyl ether; and biotin.

Examples of the amino acids include glycine, valine, leucine, isoleucine, serine, threonine, phenylaranine, alginine, lysine, aspartic acid, glutamic acid, cystine, cysteine, methionine, and tryptophan; examples of the nucleic acids include deoxyribonucleic acid; and examples of the hormones include estradiol and ethenyl estradiol.

Examples of the polymers for hair setting include amphoteric, anionic, cationic, and nonionic polymers, such as polymers of polyvinyl pyrrolidone type such as polyvinyl pyrrolidone, vinyl pyrrolidone /vinyl acetate copolymers; acidic polymers of vinyl acetate ether type such as methyl vinyl ether / maleic acid anhydride alkyl half ester copolymer; polymers of acidic poly vinyl acetate type such as vinyl acetate / crotonic acid copolymer; acidic acrylic polymers such as (meth)acrylic acid / alkyl (meth) acrylate copolymer, (meth)acrylic acid / alkyl (meth) acrylate / alkyl acrylic amide copolymer, and amphoteric acrylic polymer such as N-methacryloylethyl-N,N-dimethylammonium alpha-N-methylcarboxybetaine / alkylmetahcrylate copolymer, hydroxypropyl (meth)acrylate/butylaminoethyl methacrylate /octyl amide of acrylic acid copolymer. Use is also made of naturally occurring polymers such as cellulose or derivatives thereof, keratin, collagen and derivatives thereof.

The cosmetic as used herein include skin care cosmetics, such as milky lotion, cream, face cleansing cream, pack, massage materials, beautifying lotion, beautifying oil, detergent, deodorant, hand cream, lip cream, and wrinkle cover; makeup cosmetics such as makeup base, concealer, face powder, liquid foundation, oil foundation, rouge, eye shadow, mascara, eyeliner and lipstick; hair cosmetics such as shampoo, rinse, treatment, and setting agent; antiperspirant, and UV protective cosmetics, such as sunscreen milky lotion or sunscreen cream.

Further, the present cosmetic may be in various forms such as liquid, milky lotion, cream, emulsion, solid, paste, gel, powder, pressed, laminate, mousse, spray, and stick.

The cosmetic can be in various forms, for example, aqueous, oily, oil-in-water(O/W) type emulsion, water-in-oil(W/O) emulsion, nonaqueous emulsion, and multi-emulsion such as W/O/W or O/W/O emulsion.

### EXAMPLES

The present invention is explained in further detail below with reference to examples, but the present invention is in no way limited by the examples. In the following, "%" means "% by mass" unless otherwise specified, and viscosity data are those measured at 25 °C.

### Preparation Example 1

In a glass flask equipped with a stirrer, thermometer, and reflux condenser, 50.0 g of organopolysiloxane represented by the formula (11) shown below, 5.0g of 2-(2'-hydroxyl-5'-methacryloyloxyphenyl)-2H-benzotriazol, 35.0g of methyl methacrylate, 5.0g of butyl methacrylate, 5.0g of 2-ethylhexyl acrylate, 120.0g of toluene, and 1.0g of dimethyl-2,2'-azobis(2-methyl propionate) were placed, and subjected to polymerization by heating at a temperature of 80 °C under nitrogen gas flow for 10 hours. Then, volatiles were removed by vacuum distillation, whereby a solid silicone polymer was obtained. The polymer was dissolved at a concentration of 30% in decamethylcyclopentasiloxane (D5). The solution thus obtained is hereinafter referred to as "D5 solution 1."

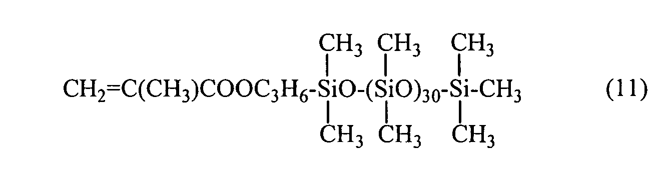

### Preparation Example 2

A solid silicone polymer was prepared in the same manner as in Preparation Example 1 except that, as a benzotriazol compound, 5.0g of 2-(2'-hydroxyl-5'-(2''-methacryloyloxyethoxy)-3'-tert-butylphenyl)-5-methl-2H-benzotriazol was used. The polymer was dissolved at a concentration of 30% in decamethylcyclopentasiloxane (D5). The solution thus obtained is hereinafter referred to as "D5 solution 2."

### Preparation Example 3

In a glass flask equipped with a stirrer, thermometer, and reflux condenser, 50.0 g of organopolysiloxane represented by the formula (12) shown below, and 30.0 g of toluene were placed and heated at a temperature of 80 °C under nitrogen gas flow. Into the glass flask, a mixed solution of 20.0 g of 2-(2'-hydroxyl-5'-methacryloyloxyphenyl)-2H-benzotriazol, 30.0g of methyl methacrylate, 120.0g of toluene, and 1.0 g of dimethyl-2,2'-azobis(2-methyl propionate) was added dropwise in 2 hours. After carrying out polymerization for 10 hours at 80 °C, volatiles were removed by vacuum distillation. A gummy silicone polymer thus obtained was dissolved at a concentration of 50% in tris(trimethylsiloxy)methylsilane (M3T). The clear pasty solution thus obtained is hereinafter referred to as "M3T solution 1."

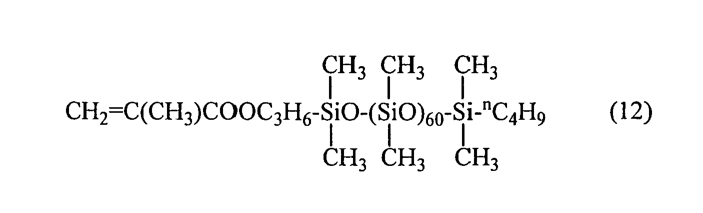

### Example 1 and Comparative Examples 1 to 3

According to the formulation shown in the following Table 1, W/O shaking type milky lotion was prepared and evaluated according the after mentioned methods. In Table 1, "Ex." stands for example and "Comp.Ex." for comparative example.

**Table 1**

| Components | | Ex.1 (%) | Comp. Ex.1 (%) | Comp. Ex.2 (%) | Comp. Ex.3 (%) |
|---|---|---|---|---|---|
| 1 | Branched polyether-modified silicone¹⁾ | 2 | 2 | 2 | 2 |
| 2 | decamethylcyclopentasiloxane | 5 | 48 | 7 | 49 |
| 3 | D5 solution 2 obtained in Preparation Example 2 | 63 | 0 | 0 | 0 |
| 4 | Octyl methoxy cinnamate | 2 | 2 | 2 | 2 |
| 5 | Glyceryl isostearate | 0 | 20 | 0 | 0 |
| 6 | Acrylic silicone resin²⁾ | 0 | 0 | 60 | 0 |
| 7 | 2-(2-hydroxyl-5-methlphenyl)-benzotriazol | 0 | 0 | 1 | 0 |
| 8 | Benzotriazol-modified silicone³⁾ | 0 | 0 | 0 | 19 |
| 9 | 1,3-butylene glycol | 3 | 3 | 3 | 3 |
| 10 | Ethanol | 5 | 5 | 5 | 5 |
| 11 | Sodium citrate | 0.2 | 0.2 | 0.2 | 0.2 |
| 12 | Purified water | 19.8 | 19.8 | 19.8 | 19.8 |
| | Evaluation Results | | | | |
| During application | | | | | |
| | Non-greasiness | A | A | C | A |
| | Spreadability | A | A | B | B |
| | Non-tackiness to the touch | A | B | B | C |
| | Smoothness | A | B | C | B |
| | Affinity for the skin | A | C | C | C |
| After applied | | | | | |
| | Non-tackiness to the touch | A | C | B | C |
| | Moisturized feel | A | B | C | C |
| | UV protective effect | A | D | D | B |

| | | | | | |
|---|---|---|---|---|---|
| 1)KF-6028 from Shin-Etsu Chemical Co.,Ltd. 2)KP-545 from Shin-Etsu Chemical Co., Ltd. 3) Silicone having a benzotriazolyl group represented by the following formula was synthesized according to the method described in J.P.No.2-282319A. 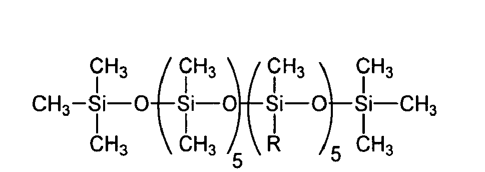 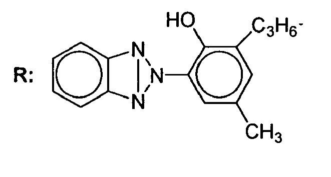 | | | | | |

### Preparation procedures

A: Components 1 to 8 were mixed.
B: To the homogeneous mixture prepared in step A, a mixture of Components 9 to 12 was added and emulsified, whereby the shaking type milky lotion was obtained.

The milky lotion thus prepared was evaluated by 30 women panelists according to the following method: An aliquot of the milky lotion was applied on the back of the right hand. During the application of the milky lotion, sensory feel was rated in terms of non-greasiness or silky feel, spreadability, non-tackiness to the touch, smoothness, and affinity for the skin. After applied, the lotion was rated in terms of non-tackiness to the touch, moisturized feel and UV protective effect. The rating was performed on a scale of 1 to 5 points as shown below, and then the points were average. UV protective effect was evaluated by applying the lotion consecutively for two weeks and then comparing degree of suntan of the back of right hand with that of the left hand.

| | |
|---|---|
| 5 points | Very good |
| 4 points | Good |
| 3 points | Average |
| 2 points | Slightly bad |
| 1 point | Bad |

| Averaged points | Overall rating |
|---|---|
| 4.5 or higher | A |
| 3.5 or higher to lower than 4.5 | B |
| 2.5 or higher to lower than 3.5 | C |
| lower than 2.5 | D |

As shown in Table 1, the lotion of Comparative Example 1 showed tackiness during and after application, and poor UV protection. The lotion of Comparative Example 2 showed bad sensory feel caused by 2-(2-hydroxyl-5-methylphenyl)benzotriazol sedimented in the lotion due to poor solubility in decamethylcyclopentasiloxane and water. The lotion of Comparative Example 3 contained nearly as much benzotriazolyl groups, which are bonded to a silicone, as that in the lotion of Example 1. However, it was tacky and has low affinity for the skin, so that UV protective effect was not durable. In contrast, the shaking type milky lotion of the Example 1 showed good sensory feel in addition to high UV protective effect.

### Example 2:W/O type milky lotion

| Components | | Mass % |
|---|---|---|
| 1. | Crosslinked polyether-modified silicone ¹⁾ | 5.0 |
| 2. | Crosslinked dimethylpolysiloxane ²⁾ | 5.0 |
| 3. | Polyether-modified silicone ³⁾ | 1.0 |
| 4. | Dimethylpolysiloxane (6 mm²/sec at 25 °C) | 8.0 |
| 5. | Decamethylcyclopentasiloxane | 10.0 |
| 6. | D5 solution 1 prepared in Preparation Example 1 | 3.0 |
| 7. | Octyl methoxy cinnamate | 2.0 |
| 8. | Glyceryl trioctanoate | 5.0 |
| 9. | 1,3-butyleneglycol | 6.0 |
| 10. | Antiseptics | q.s. |
| 11. | Perfume | q.s. |
| 12. | Purified water | 55.0 |

| | | |
|---|---|---|
| 1) KSG-210 from Shin-Etsu Chemical Co., Ltd. 2) KSG-15 from Shin-Etsu Chemical Co., Ltd. 3) KF-6017 from Shin-Etsu Chemical Co., Ltd. | | |

### Preparation procedures

A: Components 1 to 8 were mixed.
B: To the homogeneous mixture prepared in step A, a mixture of Components 9 to 12 was added and emulsified.

The milky lotion thus obtained showed non-tackiness to the touch, good spreadability, durability, affinity for the skin and stability in addition to UV-protective effect.

### Example 3:W/O type cream

| Components | | Mass % |
|---|---|---|
| 1. | Crosslinked alkyl/polyether co-modified silicone¹⁾ | 4.0 |
| 2. | Crosslinked dimethylpolysiloxane ²⁾ | 5.0 |
| 3. | Branched alkyl/polyether co-modified silicone ³⁾ | 1.5 |
| 4. | Liquid paraffin | 8.5 |
| 5. | Macadamia nut oil | 3.0 |
| 6. | M3T solution 1 obtained in Preparation Example 3 | 3.0 |
| 7. | 2,4-dihydroxybenzophenone | 2.0 |
| 8. | Pentyleneglycol | 7.0 |
| 9. | Glycerin | 3.0 |
| 10. | Sodium citrate | 0.2 |
| 11. | Antiseptics | q.s. |
| 12. | Perfume | q.s. |
| 13. | Purified water | 62.8 |

| | | |
|---|---|---|
| 1) KSG-310 from Shin-Etsu Chemical Co., Ltd. 2) KSG-15 from Shin-Etsu Chemical Co., Ltd. 3) KF-6038 from Shin-Etsu Chemical Co., Ltd. | | |

### Preparation procedures

A: Components 1 to 7 were mixed.
B: To the homogeneous mixture prepared in step A, a mixture of Components 8 to 13 was added and emulsified.

The W/O-type cream thus obtained showed non-greasiness, refreshing feel, non-tackiness to the touch, good spreadability, durability, affinity for the skin and glossy finish in addition to UV-protective effect.

### Example 4:W/O type cream

| Components | | Mass % |
|---|---|---|
| 1. | Paste composition¹⁾ | 7.0 |
| 2. | Crosslinked alkyl-modified dimethylpolysiloxane²⁾ | 5.0 |
| 3. | Branched alkyl/polyether co-modified silicone³⁾ | 0.5 |
| 4. | Isododecane | 5.0 |
| 5. | D5 solution 1 obtained in Preparation Example 1 | 0.5 |
| 6. | Butylmethoxydibenzoylmethane | 3.0 |
| 7. | Composite powder of hybrid silicone⁴⁾ | 2.0 |
| 8. | Sodium citrate | 0.2 |
| 9. | Propylene glycol | 8.0 |
| 10. | Glycerin | 3.0 |
| 11. | Antiseptics | q.s. |
| 12. | Perfume | q.s. |
| 13. | Purified water | 65.8 |

| | | |
|---|---|---|
| 1) The paste composition prepared by kneading 30 parts by mass of POE-modified crosslinked silicone with 70 parts by weight of lauroyl sarcosinate isopropyl oil. 2) KSG-42 from Shin-Etsu Chemical Co., Ltd. 3) KF-6038 from Shin-Etsu Chemical Co., Ltd 4) KSP-100 from Shin-Etsu Chemical Co., Ltd. | | |

### Preparation procedures

A: Components 1 to 7 were mixed.
B: To the homogeneous mixture prepared in step A, a mixture of Components 8 to 13 was added and emulsified.

The W/O-type cream thus obtained showed non-greasiness, non-tackiness to the touch, good spreadability, durability, affinity for the skin and matt finish in addition to UV-protective effect.

### Example 5:O/W type cream

| Components | | Mass % |
|---|---|---|
| 1. | Crosslinked dimethylpolysiloxane¹⁾ | 10.0 |
| 2. | Isotridecyl isononanate | 5.0 |
| 3. | D5 solution 2 obtained in Preparation Example 2 | 5.0 |
| 4. | Octyl methoxy cinnamate | 2.0 |
| 5. | Polyether-modified silicone²⁾ | 0.5 |
| 6. | Dipropylene glycol | 6.0 |
| 7. | Glycerin | 5.0 |
| 8. | Methylcellulose(2% aqueous solution)³⁾ | 5.0 |
| 9. | Polyacrylamide emulsifier⁴⁾ | 2.0 |
| 10. | Antiseptics | q.s. |
| 11. | Perfume | q.s. |
| 12. | Purified water | 59.5 |

| | | |
|---|---|---|
| 1) KSG-15 from Shin-Etsu Chemical Co., Ltd. 2) KF-6011 from Shin-Etsu Chemical Co., Ltd. 3) Metholose SM-4000 from Shin-Etsu Chemical Co., Ltd. 4) Sepigel 305 from Seppic. Co. | | |

### Preparation procedures

A: Components 6 to 12 were mixed.
B: To the homogeneous mixture prepared in step A, a mixture of Components 1 to 5 was added and emulsified.

The O/W-type cream thus obtained showed non-greasiness, non-tackiness to the touch, good spreadability, moisturized feel, and stability with time and temperature in addition to UV-protective effect.

### Example 6:O/W type cream

| Components | | Mass % |
|---|---|---|
| 1. | Crosslinked dimethylpolysiloxane¹⁾ | 10.0 |
| 2. | Crosslinked dimethylpolysiloxane ²⁾ | 20.0 |
| 3. | Isotridecyl isononanate | 7.5 |
| 4. | M3T solution 1 obtained in Preparation Example 3 | 2.5 |
| 5. | Octyl methoxy cinnamate | 1.5 |
| 6. | Branched polyglycerin-modified silicone³⁾ | 0.5 |
| 7. | Branched polyglycerin-modified silicone⁴⁾ | 0.5 |
| 8. | Dipropylene glycol | 6.0 |
| 9. | Glycerin | 5.0 |
| 10. | Polyacrylamide emulsifier⁵⁾ | 2.0 |
| 11. | Antiseptics | q.s. |
| 12. | Perfume | q.s. |

| | | |
|---|---|---|
| 1) KSG-15 from Shin-Etsu Chemical Co., Ltd. 2) KSG-16 from Shin-Etsu Chemical Co., Ltd. 3) KF-6100 from Shin-Etsu Chemical Co., Ltd. 4) KF-6104 from Shin-Etsu Chemical Co., Ltd. 5) Simulgel 600 from Seppic.Co. | | |

### Preparation procedures

A: Components 6 to 13 were mixed.
B: To the homogeneous mixture prepared in step A, a mixture of Components 1 to 5 was added and emulsified.

The O/W-type cream thus obtained showed fine texture, smooth spreadability, non-greasiness, non-tackiness to the touch, moisturized feel, and stability with time and temperature in addition to UV-protective effect.

### Example 7:W/O type cream

| Components | | Mass % |
|---|---|---|
| 1. | D5 solution 1 obtained in Preparation Example 1 | 7.0 |
| 2. | Dimethylpolysiloxane (6 mm²/sec at 25 °C) | 10.0 |
| 3. | Branched polyether-modified silicone¹⁾ | 1.5 |
| 4. | Dispersion of titanium oxide in cyclopentasiloxane²⁾ | 5.0 |
| 5. | Dispersion of zinc oxide in cyclopentasiloxane³⁾ | 5.0 |
| 6. | Dipropylene glycol | 10.0 |
| 7. | Sodium citrate | 0.2 |
| 8. | Ethanol | 5.0 |
| 9. | Antiseptics | q.s. |
| 10. | Perfume | q.s. |
| 11. | Purified water | 56.3 |

| | | |
|---|---|---|
| 1) KF-6028 from Shin-Etsu Chemical Co.,Ltd. 2) SPD-T5 from Shin-Etsu Chemical Co., Ltd. 3) SPD-Z5 from Shin-Etsu Chemical Co., Ltd. | | |

### Preparation procedures

A: Components 6 to 13 were mixed.
B: To the homogeneous mixture prepared in step A, a mixture of Components 1 to 5 was added and emulsified.

The O/W-type cream thus obtained showed fine texture, smooth spreadability, non-greasiness, non-tackiness to the touch, moisturized feel, and stability with time and temperature in addition to UV-protective effect.

### Example 8:W/O type makeup base

| Components | | Mass % |
|---|---|---|
| 1. | Crosslinked polyglycerin-modified silicone¹⁾ | 5.0 |
| 2. | Crosslinked dimethylpolysiloxane ²⁾ | 1.0 |
| 3. | Branched polyglycerin-modified silicone³⁾ | 0.5 |
| 4. | Dimethylpolysiloxane (6 mm²/sec at 25 °C) | 6.0 |
| 5. | Dimethylpolysiloxane(20mm²/sec at 25 °C) | 2.0 |
| 6. | D5 solution 2 obtained in Preparation Example 2 | 3.0 |
| 7. | Dispersion of titanium oxide in cyclopentasiloxane⁴⁾ | 10.0 |
| 8. | Dipropylene glycol | 5.0 |
| 9. | Sodium citrate | 0.2 |
| 10. | Methylcellulose(2% aqueous solution)⁵⁾ | 2.5 |
| 11. | Ethanol | 3.0 |
| 12. | Antiseptics | q.s. |
| 13. | Perfume | q.s. |
| 14. | Purified water | 61.8 |

| | | |
|---|---|---|
| 1) KSG-710 from Shin-Etsu Chemical Co., Ltd. 2) KSG-15 from Shin-Etsu Chemical Co., Ltd. 3) KF-6104 from Shin-Etsu Chemical Co., Ltd. 4) SPD-T5 from Shin-Etsu Chemical Co., Ltd. 5) Metholose 65-SH4000 from Shin-Etsu Chemical Co., Ltd. | | |

### Preparation procedures

A: Components 1 to 7 were mixed.
B: To the homogeneous mixture prepared in step A, a mixture of Components 8 to 15 was added and emulsified.

The makeup base thus obtained showed non-greasiness, non-tackiness to the touch, smooth spreadability, moisturized feel, durability, affinity for the skin, and matt finish in addition to UV-protective effect.

### Example 9:O/W type cream

| Components | | Mass % |
|---|---|---|
| 1. | Crosslinked alkyl-modified dimethylpolysiloxane¹⁾ | 7.0 |
| 2. | Crosslinked dimethylpolysiloxane ²⁾ | 10.0 |
| 3. | Decamethylcyclopentasiloxane | 10.0 |
| 4. | Dimethylpolysiloxane (6 mm²/sec at 25 °C) | 10.0 |
| 5. | M3T solution 1 obtained in Preparation Example 3 | 8.0 |
| 6. | Methyl anthranilate | 2.0 |
| 7. | Polyether-modified silicone ³⁾ | 0.7 |
| 8. | Propylene glycol | 3.0 |
| 9. | Mixture of polyacrylamide⁴⁾ | 0.8 |
| 10. | Xanthan gum (2% aqueous solution) | 8.0 |
| 11. | Antiseptics | q.s. |
| 12. | Perfume | q.s. |
| 13. | Purified water | 40.5 |

| | | |
|---|---|---|
| 1) KSG-43 from Shin-Etsu Chemical Co., Ltd. 2) KSG-16 from Shin-Etsu Chemical Co., Ltd. 3) KF-6011 from Shin-Etsu Chemical Co., Ltd. 4) Sepigel 305 from Seppic Co. | | |

### Preparation procedures

A: Components 1 to 6 were heat-mixed.
B: Components 7 to 13 were mixed.
C: To the homogeneous mixture prepared in step B, mixture prepared in step A was added and emulsified.

The cream thus obtained showed fine texture, good spreadability, non-greasiness, moisturized and refreshing feel, durability, and stability with time and temperature in addition to UV-protective effect.

### Example 10:Lipstick

| Components | | Mass % |
|---|---|---|
| 1. | Polyethylene wax | 12.0 |
| 2. | Microcrystalline wax | 4.0 |
| 3. | Polybutene | 5.0 |
| 4. | Stearylacrylate/dimethylsilicone copolymer¹⁾ | 12.0 |
| 5. | D5 solution 1 obtained in Preparation Example 1 | 7.0 |
| 6. | Octyl methoxy cinnamate | 2.0 |
| 7. | Cetyl isooctanoate | 20.0 |
| 8. | Sucrose fatty acid esters | 3.0 |
| 9. | Glyceryl triisostearate | 35.0 |
| 10. | Pigment | q.s. |
| 11. | Antiseptics | q.s. |
| 12. | Perfume | q.s. |

| | | |
|---|---|---|
| 1) KP-561P from Shin-Etsu Chemical Co., Ltd. | | |

### Preparation procedures

A: Components 1 to 8 and a part of Component 9 were heat-mixed.
B: Components 10 to 12 and the rest of Component 9 were mixed. The mixture obtained was added to the mixture prepared in step A. The mixture obtained was poured in a container, and a lipstick was obtained.

The lipstick thus obtained showed good spreadability, non-greasiness, non-powdery texture, refreshing feel, and water-repellency in addition to UV-protective effect.

### Example 11:Powder foundation

| Components | | Mass % |
|---|---|---|
| 1. | D5 solution 2 obtained in Preparation Example 2 | 5.0 |
| 2. | Squalane | 1.0 |
| 3. | Crosslinked dimethylpolysiloxane¹⁾ | 2.5 |
| 4. | Glyceryl trioctanoate | 2.0 |
| 5. | Mica treated with silicone | 40.0 |
| 6. | Talc treated with silicone | Balance |
| 7. | Titanium oxide treated with silicone | 10.0 |
| 8. | Titanium oxide fine powder treated with silicone | 5.0 |
| 9. | Barium sulfate treated with silicone | 10.0 |
| 10. | Pigment | q.s. |
| 11. | Composite powder of phenyl-modified hybrid silicone²⁾ | 2.0 |
| 12. | Polymethylsilsesquioxane spherical powder³⁾ | 2.5 |
| 13. | Antiseptics | q.s. |
| 14. | Perfume | q.s. |

| | | |
|---|---|---|
| 1) KSG-16 from Shin-Etsu Chemical Co., Ltd. 2) KSP-300 from Shin-Etsu Chemical Co., Ltd. 3) KMP-590 from Shin-Etsu Chemical Co., Ltd. | | |

### Preparation procedures

A: Components 5 to 13 were mixed.
B: To the homogeneous mixture prepared in the step A, a mixture of Components 1 to 4 were added and mixed.
C: To the homogeneous mixture prepared in the step B, Component 14 was added and mixed. The mixture obtained was placed in a metal mold and pressed into a powder foundation.

The powder foundation thus obtained showed non-tackiness to the touch, good spreadability, lasted long on the skin, and had affinity for the skin to give matt finish in addition to UV-protective effect.

### Example 12:Cream foundation

| Components | | Mass % |
|---|---|---|
| 1. | M3T solution 1 obtained in Preparation Example 3 | 5.0 |
| 2. | 4-t-butyl-4'-methoxybenzoylmethane | 1.0 |
| 3. | Glyceryl trioctanoate | 4.0 |
| 4. | Dimethylpolysiloxane (6 mm²/sec at 25 °C) | 5.0 |
| 5. | Methyltrimethicone | 6.5 |
| 6. | Composite powder of phenyl-modified hybrid silicone¹⁾ | 2.5 |
| 7. | Pigment | 8.0 |
| 8. | Acrylic silicone resin²⁾ | 5.0 |
| 9. | Dipropylene glycol | 5.0 |
| 10. | Sodium citrate | 0.2 |
| 11. | Antiseptics | q.s. |
| 12. | Perfume | q.s. |
| 13. | Purified water | 57.8 |

| | | |
|---|---|---|
| 1) KSP-300 from Shin-Etsu Chemical Co., Ltd. 2) KMP-575 from Shin-Etsu Chemical Co., Ltd. | | |

### Preparation procedures

A: Components 1 to 6 were heat-mixed.
B: To the homogeneous mixture prepared in the step A, a mixture of Components 9 to 13 were added and emulsified.
C: To the emulsion prepared in the step B, a mixture of Components 7 and 8 was added.

The cream foundation thus obtained showed non-tackiness to the touch, good spreadability, lasted long on the skin, and had affinity for the skin to give matt finish, in addition to UV-protective effect.

### Example 13:W/O type compact foundation

| Components | | Mass % |
|---|---|---|
| 1. | Ceresin wax | 5.5 |
| 2. | Microcrystalline wax | 1.0 |
| 3. | Liquid paraffin | 3.0 |
| 4. | D5 solution 1 obtained in Preparation Example 1 | 9.0 |
| 5. | Polypropylene glycol dicaprirate | 3.0 |
| 6. | Branched alkyl/polyether-modified silicone¹⁾ | 1.0 |
| 7. | Dimethylpolysiloxane (6 mm²/sec at 25 °C) | 15.5 |
| 8. | Titanium oxide treated with oil | 10.0 |
| 9. | Pigment | q.s. |
| 10. | Lecithin | 0.3 |
| 11. | Polyoxyethylene sorbitan monooleate | 0.5 |
| 12. | Dipropylene glycol | 8.0 |
| 13. | Sodium citrate | 0.2 |
| 14. | Purified water | Balance |

| | | |
|---|---|---|
| 1) KF-6038 from Shin-Etsu Chemical Co., Ltd. | | |

### Preparation procedures

A: Components 1 to 7 were heat-mixed.
B: Components 8 to 12 were mixed
C: To the homogeneous mixture prepared in the step B, a mixture of Components 13 and 14 was added and heated.
D: The mixture prepared in the step C was added to the mixture prepared in the step A.

The compact foundation thus obtained showed non-greasiness, even with high contents of unctuous agents. It was non-tackiness to the touch, showed good spreadability, lasted long on the skin, and had affinity for the skin in addition to UV-protective effect.

### Example 14:Powder foundation

| Components | | Mass % |
|---|---|---|
| 1. | Crosslinked polyether-modified silicone ¹⁾ | 5.0 |
| 2. | Squalane | 1.0 |
| 3. | Glyceryl trioctanoate | 2.0 |
| 4. | Mica treated with the D5 solution 2²⁾ | 40.0 |
| 5. | Talc treated with silicone³⁾ | Balance |
| 6. | Titanium oxide treated with silicone³⁾ | 10.0 |
| 7. | Barium sulfate treated with silicone³⁾ | 10.0 |
| 8. | Pigment treated with silicone³⁾ | q.s. |
| 9. | Composite powder of hybrid silicone⁴⁾ | 2.0 |
| 10. | Polymethylsilsesquioxane spherical powder⁵⁾ | 2.5 |
| 11. | Antiseptics | q.s. |
| 12. | Perfume | q.s. |

| | | |
|---|---|---|
| 1) KSG-210 from Shin-Etsu Chemical Co., Ltd. 2) Mica was heat-treated with 10 g of D5 solution 2 prepared in Preparation Example 2 and then dried. 3) Powder treated with KF-9909 from Shin-Etsu Chemical Co., Ltd. 4) KSP-100 from Shin-Etsu Chemical Co., Ltd. 5) KMP-590 from Shin-Etsu Chemical Co., Ltd. | | |

### Preparation procedures

A: Components 5 to 13 were mixed
B: Components 1 to 4 were mixed and added to the mixture prepared in the step A.
C: To the homogeneous mixture prepared in the step B, Component 14 was added and mixed. The mixture obtained was placed in a mold and pressed.

The powder foundation thus obtained showed non-tackiness to the touch, good spreadability. It lasted long on the skin, and had affinity for the skin to give glossy finish in addition to UV-protective effect.

### Example 15:Eye shadow

| Components | | Mass % |
|---|---|---|
| 1. | Sericite treated with acryl-modified silicone¹⁾ | 40.0 |
| 2. | Mica treated with acryl-modified silicone¹⁾ | 10.0 |
| 3. | Talc treated with acryl-modified silicone¹⁾ | Balance |
| 4. | Titanium oxide treated with acryl-modified silicone¹⁾ | 10.0 |
| 5. | Magnesium stearate | 3.0 |
| 6. | Pigment | q.s. |
| 7. | Octyl dodecanol | 3.0 |
| 8. | M3T solution 1 obtained in Preparation Example 3 | 4.0 |
| 9. | Crosslinked alkyl-modified dimethylpolysiloxane²⁾ | 6.0 |
| 10. | Antiseptics | q.s. |
| 11. | Perfume | q.s. |

| | | |
|---|---|---|
| 1) KMP-574 treated powder from Shin-Etsu Chemical Co., Ltd. 2) KSG-43 from Shin-Etsu Chemical Co., Ltd. | | |

### Preparation procedures

A: Components 7 to 10 were heat-mixed.
B: Components 1 to 6 were mixed and added to the mixture prepared in the step A.
C: To the homogeneous mixture prepared in the step B, Component 11 was added.

The eye shadow thus obtained showed non-tackiness to the touch, good spreadability. It lasted long on the skin, gave moisturized feel and had affinity for the skin to give glossy finish in addition to UV-protective effect.

### Example 16: Powder eyebrow

| Components | | Mass % |
|---|---|---|
| 1. | Vaseline | 2.5 |
| 2. | Dimethylpolysiloxane (6 mm²/sec at 25 °C) | 1.5 |
| 3. | Crosslinked alkyl-modified dimethylpolysiloxane¹⁾ | 2.5 |
| 4. | Glyceryl trioctanoate | 2.0 |
| 5. | Mica treated with the D5 solution 2²⁾ | 40.0 |
| 6. | Talc treated with the D5 solution 2³⁾ | Balance |
| 7. | Titanium oxide treated with silicone⁴⁾ | 10.0 |
| 8. | Barium sulfate treated with silicone⁴⁾ | 15.0 |
| 9. | Pigment treated with silicone⁴⁾ | q.s. |
| 10. | Composite powder of phenyl-modified hybrid silicone⁵⁾ | 1.5 |
| 11. | Polyamide spherical powder | 2.5 |
| 12. | Antiseptics | q.s. |
| 13. | Perfume | q.s. |

| | | |
|---|---|---|
| 1) KSG-43 from Shin-Etsu Chemical Co., Ltd. 2) Mica was treated with 10 g of D5 solution 2 prepared in Synthesis Example 2 by heating and then dried. 3) Talc was treated with 10 g of D5 solution 2 prepared in Synthesis Example 2 by heating and then dried. 4) KF-9908 treated powder from Shin-Etsu Chemical Co., Ltd. 5) KSP-300 from Shin-Etsu Chemical Co., Ltd. | | |

### Preparation procedures

A: Components 5 to 12 were mixed.
B: Components 1 to 4 were mixed and added to the homogeneous mixture prepared in the step A.
C: To the homogeneous mixture prepared in the step B, Component 13 was added. The mixture obtained was placed in a metal mold and pressed, whereby powder eyebrow was obtained.

The eye shadow thus obtained showed non-tackiness to the touch, good spreadability. It lasted long on the skin, had affinity for the skin to give glossy finish in addition to UV-protective effect.

### Example 17: Hair cream

| Components | | Mass % |
|---|---|---|
| 1. | D5 solution 1 obtained in Preparation Example 1 | 5.0 |
| 2. | Octyl methoxy cinnamate | 1.0 |
| 3. | Dimethylpolysiloxane (6 mm²/sec at 25 °C) | 5.0 |
| 4. | Decamethylcyclopentasiloxane | 5.0 |
| 5. | Stearyltrimethylammonium chloride | 1.5 |
| 6. | Glycerin | 3.0 |
| 7. | Propylene glycol | 5.0 |
| 8. | Hydroxyethylcellulose | 0.2 |
| 9. | Antiseptics | q.s. |
| 10. | Perfume | q.s. |
| 11. | Purified water | 74.3 |

### Preparation procedures

A: Components 1 to 4 were mixed.
B: Components 5 to 9 and 11 were mixed.
C: The solution prepared in the step B was added to the mixture obtained in the step A and emulsified. The emulsion was cooled, to which Component 10 was added.

The hair cream thus obtained spread well on the hair. It provided to the hair with softness, smoothness, moisturized feel, settability and gloss finish in addition to UV-protective effect.

### Example 18: Conditioning mousse

| Components | | Mass % |
|---|---|---|
| 1. | D5 solution 2 obtained in Preparation Example 2 | 5.0 |
| 2. | Octyl methoxy cinnamate | 1.0 |
| 3. | Dimethylpolysiloxane (6 mm²/sec at 25 °C) | 1.0 |
| 4. | Crosslinked dimethylpolysiloxane¹⁾ | 0.5 |
| 5. | Glyceryl trioctanoate | 1.5 |
| 6. | Glycerin | 3.0 |
| 7. | Stearyldimethylbenzylammonium chloride | 0.5 |
| 8. | Polyoxyethylene hydrogenated castor oil | 0.5 |
| 9. | Ethanol | 7.0 |
| 10. | Antiseptics | q.s. |
| 11. | Perfume | q.s. |

| | | |
|---|---|---|
| 1) KSG-16 from Shin-Etsu Chemical Co., Ltd. | | |

### Preparation procedures

A: Components 1 to 5 were mixed.
B: Components 6 to 10 and 12 were mixed.
C: The solution prepared in the step B was added to the mixture obtained in the step A and emulsified. The emulsion was cooled, to which Component 11 was added.
D: The mixture prepared in the step C and Component 13 were put in an aerosol can, whereby a conditioning mousse was obtained.

The conditioning mousse thus obtained provided to the hair with softness, smoothness, moisturized feel, non-greasiness, and matt finish in addition to UV-protective effect.

### Example 19:Roll-on type antiperspirant

| Components | | Mass % |
|---|---|---|
| 1. | M3T solution 1 obtained in Preparation Example 3 | 15.0 |
| 2. | Octyl methoxy cinnamate | 2.0 |
| 3. | Dimethylpolysiloxane (6 mm²/sec at 25 °C) | 10.0 |
| 4. | Crosslinked dimethylpolysiloxane¹⁾ | 23.0 |
| 5. | Decamethylcyclopentasiloxane | 25.0 |
| 6. | Composite powder of hybrid silicone²⁾ | 5.0 |
| 7. | Aluminum/zirconium tetrachlorohydrate | 20.0 |
| 8. | Perfume | q.s. |

| | | |
|---|---|---|
| 1) KSG-15 from Shin-Etsu Chemical Co., Ltd. 2) KSP-100 from Shin-Etsu Chemical Co., Ltd. | | |

### Preparation procedures

A: Components 1 to 6 were mixed.
B: In the mixture prepared in the step A, Components 7 and 8 were dispersed.

The roll-on type antiperspirant thus obtained showed good spreadability, coolness, refreshing feel, non-greasiness, and stability with time and temperature in addition to UV-protective effect.

### Example 20:W/O type antiperspirant

| Components | | Mass % |
|---|---|---|
| 1. | Crosslinked polyether-modified silicone ¹⁾ | 4.0 |
| 2. | D5 solution 1 obtained in Preparation Example 1 | 8.0 |
| 3. | Octyl methoxy cinnamate | 1.0 |
| 4. | Decamethylcyclopentasiloxane | 8.0 |
| 5. | Glyceryl trioctanoate | 4.0 |
| 6. | 1,3-butylene glycol | 5.0 |
| 7. | Sodium citrate | 0.2 |
| 8. | Aluminum chlorohydrate | 20.0 |
| 9. | Perfume | q.s. |
| 10. | Purified water | 49.8 |

| | | |
|---|---|---|
| 1) KSG-210 from Shin-Etsu Chemical Co., Ltd. | | |

### Preparation procedures

A: Components 1 to 5 were mixed.
B: Components 6,7 and 10 were mixed, to which Components 8 and 9 were added and dissolved.
C: To the mixture prepared in the step A, the solution prepared in step B was added and emulsified by stirring.

The antiperspirant thus obtained showed good spreadability, coolness, refreshing feel, non-greasiness, non-tackiness to the touch and stability with time and temperature in addition to UV-protective effect.

### Example 21:W/O type UV cut-off cream

| Components | | Mass % |
|---|---|---|
| 1. | Zinc oxide treated with a silicone¹⁾ | 20.0 |
| 2. | Acrylate/dimethylsilicone copolymer²⁾ | 7.0 |
| 3. | Decamethylcyclopentasiloxane | 10.0 |
| 4. | Glyceryl trioctanoate | 3.0 |
| 5. | D5 solution 2 obtained in Preparation Example 2 | 7.0 |
| 6. | Polyether-modified silicone ³⁾ | 1.0 |
| 7. | Branched alkyl/polyglycerin-modified silicone⁴⁾ | 1.0 |
| 8. | Octyl methoxy cinnamate | 6.0 |
| 9. | Sodium citrate | 0.2 |
| 10. | Dipentylene glycol | 4.0 |
| 11. | Antiseptics | q.s. |
| 12. | Perfume | q.s. |
| 13. | Purified water | 40.8 |

| | | |
|---|---|---|
| 1) Zinc oxide treated with KF-9901from Shin-Etsu Chemical Co., Ltd. 2) KP-545 from Shin-Etsu Chemical Co., Ltd. 3) KF-6017 from Shin-Etsu Chemical Co., Ltd. 4) KF-6105 from Shin-Etsu Chemical Co., Ltd. | | |

### Preparation procedures

A: Components 4 to 8 and a part of Component 3 were mixed.
B: Components 9 to 11 and 13 were mixed, and the mixture obtained was added to the mixture prepared in the step A and emulsified.
C: Components 1, 2 and the rest of Component 3 were mixed, to which the emulsion prepared in the step B and Component 12 were mixed.

The UV cut-off cream thus obtained showed good spreadability, refreshing feel, non-greasiness, non-tackiness to the touch and stability with time and temperature in addition to UV-protective effect.

### Example 22:W/O type UV cut-off milky lotion

| Components | | Mass % |
|---|---|---|
| 1. | Crosslinked polyether-modified silicone ¹⁾ | 3.0 |
| 2. | Crosslinked dimethylpolysiloxane ²⁾ | 2.0 |
| 3. | Dimethylpolysiloxane (6 mm²/sec at 25 °C) | 5.0 |
| 4. | Glyceryl trioctanoate | 3.0 |
| 5. | M3T solution 1 obtained in Preparation Example 3 | 6.0 |
| 6. | Branched polyether-modified silicone³⁾ | 1.0 |
| 7. | Bentonite modified with an organic compound | 0.2 |
| 8. | Titanium oxide dispersion in decamethylcyclopentasiloxane⁴⁾ | 30.0 |
| 9. | Zinc oxide dispersion in decamethylcyclopentasiloxane⁵⁾ | 30.0 |
| 10. | Dipropylene glycol | 2.0 |
| 11. | Sodium citrate | 0.2 |
| 12. | Sodium chloride | 0.5 |
| 13. | Antiseptics | q.s. |
| 14. | Perfume | q.s. |
| 15. | Purified water | 17.1 |

| | | |
|---|---|---|
| 1) KSG-210 from Shin-Etsu Chemical Co., Ltd. 2) KSG-15 from Shin-Etsu Chemical Co., Ltd. 3) KF-6028 from Shin-Etsu Chemical Co.,Ltd. 4) SPD-T6 from Shin-Etsu Chemical Co.,Ltd. 5) SPD-Z6 from Shin-Etsu Chemical Co.,Ltd. | | |

### Preparation procedures

A: Components 1 to 7 were heat-mixed.
B: Components 10 to 13 and 15 were mixed, and the mixture obtained was added to the mixture prepared in the step A and emulsified.
C: Components 8, 9 and 14 were mixed and the mixture obtained was added to the emulsion prepared in the step B.

The UV cut-off lotion thus obtained showed good spreadability, refreshing feel, non-greasiness, non-tackiness to the touch and stability with time and temperature in addition to UV-protective effect.

### Example 23:Shaking type UV cut-off lotion

| Components | | Mass % |
|---|---|---|
| 1. | Branched polyether-modified silicone¹⁾ | 2.0 |
| 2. | Decamethylcyclopentasiloxane | 8.8 |
| 3. | Octyl methoxy cinnamate | 7.5 |
| 4. | Dimethylpolysiloxane (6 mm²/sec at 25 °C) | 6.0 |
| 5. | D5 solution 1 obtained in Preparation Example 1 | 5.0 |
| 6. | Composite powder of hybrid silicone²⁾ | 0.5 |
| 7. | Clay minerals modified with an organic compound | 0.2 |
| 8. | Titanium oxide dispersion in decamet hylcyclopentasiloxane³⁾ | 1.0 |
| 9. | Zinc oxide dispersion in decamethylcyclopentasiloxane⁴⁾ | 40.0 |
| 10. | Dipentylene glycol | 3.0 |
| 11. | Sodium chloride | 0.5 |
| 12. | Ethanol | 5.0 |
| 13. | Antiseptics | q.s. |
| 14. | Perfume | q.s. |
| 15. | Purified water | 20.5 |

| | | |
|---|---|---|
| 1) Zinc oxide treated with KF-6028 from Shin-Etsu Chemical Co.,Ltd. 2) KSP-105 from Shin-Etsu Chemical Co.,Ltd. 3) SPD-T5 from Shin-Etsu Chemical Co., Ltd. 4) SPD-Z6 from Shin-Etsu Chemical Co., Ltd. | | |

### Preparation procedures

A: Components 1 to 7 and a part of Component 3 were mixed.
B: Components 10 to 13 and 15 were mixed, and the mixture obtained was added to the mixture prepared in the step A and emulsified.
C: Components 8, 9 and the rest of Component 3 were mixed. The mixture obtained and Component 14 was added to the emulsion prepared in the step B.

The shaking type UV cut-off lotion thus obtained showed good spreadability, non-greasiness, and non-tackiness to the touch in addition to UV-protective effect. It lasted long and was stable with time and temperature.

### Example 24:W/O type UV cut-off cream

| Components | | Mass % |
|---|---|---|
| 1. | Crosslinked polyglycerin-modified silicone¹⁾ | 2.0 |
| 2. | Crosslinked dimethylpolysiloxane ²⁾ | 3.0 |
| 3. | D5 solution 2 obtained in Preparation Example 2 | 2.0 |
| 4. | Neopentyl glycol dioctanoate | 1.0 |
| 5. | Branched alkyl/polyglycerin-modified silicone³⁾ | 1.0 |
| 6. | Silica | 0.2 |
| 7. | Titanium oxide dispersion in decamethylcyclopentasiloxane⁴⁾ | 25.0 |
| 8. | Zinc oxide dispersion in decamethylcyclopentasiloxane⁵⁾ | 15.0 |
| 9. | 1,3-butylene glycol | 5.0 |
| 10. | Sodium citrate | 0.2 |
| 11. | Sodium chloride | 0.5 |
| 12. | Antiseptics | q.s. |
| 13. | Perfume | q.s. |
| 14. | Purified water | 45.1 |

| | | |
|---|---|---|
| 1) KSG-710 from Shin-Etsu Chemical Co., Ltd. 2) KSG-15 from Shin-Etsu Chemical Co., Ltd. 3) KF-6105 from Shin-Etsu Chemical Co., Ltd. 4) SPD-T5 from Shin-Etsu Chemical Co., Ltd. 5) SPD-Z5 from Shin-Etsu Chemical Co., Ltd. | | |

### Preparation procedures

A: Components 1 to 8 were mixed.
B: Components 9 to 12 and 14 were mixed, and the mixture obtained was added to the mixture prepared in the step A and emulsified.
C: Component 13 was added to the emulsion prepared in the step B.

The UV cut-off cream thus obtained showed good spreadability, non-greasiness, and non-tackiness to the touch in addition to UV-protective effect. It lasted long and was stable with time and temperature.

### Example 25:O/W type UV cut-off cream

| Components | | Mass % |
|---|---|---|
| 1. | Crosslinked organopolysiloxanel¹⁾ | 5.0 |
| 2. | Cetyl isooctanoate | 5.0 |
| 3. | M3T solution 1 obtained in Preparation Example 3 | 1.0 |
| 4. | Titanium oxide dispersion in decamethylcyclopentasiloxane²⁾ | 15.0 |
| 5. | Polyether-modified silicone ³⁾ | 1.0 |
| 6. | Polyether-modified silicone⁴⁾ | 1.0 |
| 7. | Mixture of acrylamide⁵⁾ | 2.0 |
| 8. | Propylene glycol | 5.0 |
| 9. | Methylcellulose(2% aqueous solution)⁶⁾ | 5.0 |
| 10. | Antiseptics | q.s. |
| 11. | Perfume | q.s. |
| 12. | Purified water | 60.0 |

| | | |
|---|---|---|
| 1) KSG-15 from Shin-Etsu Chemical Co., Ltd. 2) SPD-T5 from Shin-Etsu Chemical Co., Ltd. 3) KF-6013 from Shin-Etsu Chemical Co., Ltd. 4) KF-6011 from Shin-Etsu Chemical Co., Ltd. 5) Sepigel 305 from Seppic Co. 6) Metholose SM-4000 from Shin-Etsu Chemical Co., Ltd. | | |

### Preparation procedures

A: Components 5 to 8, 10 and 12 were mixed.
B: Components 1 to 3 were mixed, and the mixture obtained was added to the mixture prepared in the step A and emulsified.
C: Component 4 was added to the emulsion prepared in the step B, to which Components 9 and 10 were mixed.

The UV protective cream thus obtained showed good spreadability, non-greasiness, non-tackiness to the touch, and transparent appearance in addition to UV-protective effect. It lasted long and was stable with time and temperature.

### Example 26:Non-aqueous emulsion

| Components | | Mass % |
|---|---|---|
| 1. | Crosslinked dimethylpolysiloxane¹⁾ | 30.0 |
| 2. | Decamethylcyclopentasiloxane | 15.0 |
| 3. | Dimethylpolysiloxane (6 mm²/sec at 25 °C) | 7.0 |
| 4. | D5 solution 1 obtained in Preparation Example | 1 3.0 |
| 5. | Octyl methoxy cinnamate | 2.0 |
| 6. | Dimethylstearylammonium hectorite | 2.0 |
| 7. | 1,3-butylene glycol | 41.0 |

| | | |
|---|---|---|
| 1) KSG-15 from Shin-Etsu Chemical Co., Ltd. | | |

### Preparation procedures

A: Components 1 to 6 were mixed.
B: Component 7 was added to the homogeneous mixture prepared in the step A and emulsified by stirring.

The non-aqueous emulsion thus obtained showed good spreadability, non-greasiness, non-tackiness to the touch, and moisturized feel in addition to UV-protective effect. It was stable with time and temperature.

### Example 27:Non-aqueous emulsion

| Components | | Mass % |
|---|---|---|
| 1. | Crosslinked polyglycerin-modified silicone¹⁾ | 5.0 |
| 2. | Crosslinked dimethylpolysiloxane ²⁾ | 30.0 |
| 3. | Crosslinked dimethylpolysiloxane³⁾ | 35.0 |
| 4. | Dimethylpolysiloxane (6 mm²/sec at 25 °C) | 3.0 |
| 5. | D5 solution 2 obtained in Preparation Example 2 | 6.0 |
| 6. | Octyl methoxy cinnamate | 1.0 |
| 7. | Branched polyglycerin-modified silicone⁴⁾ | 1.0 |
| 8. | Composite powder of phenyl-modified hybrid silicone⁵⁾ | 5.0 |
| 9. | Glycerin | 5.0 |
| 10. | 1,3-butylene glycol | 10.0 |

| | | |
|---|---|---|
| 1) KSG-710 from Shin-Etsu Chemical Co., Ltd. 2) KSG-15 from Shin-Etsu Chemical Co., Ltd. 3) KSG-16 from Shin-Etsu Chemical Co., Ltd. 4) KF-6104 from Shin-Etsu Chemical Co., Ltd. 5) KSP-300 from Shin-Etsu Chemical Co., Ltd. | | |

### Preparation procedures

A: Components 1 to 8 were mixed.
B: Components 9 and 10 were added to the homogeneous mixture prepared in the step A and emulsified by stirring.

The non-aqueous emulsion thus obtained showed good spreadability, non-greasiness, non-tackiness to the touch, and moisturized feel in addition to UV-protective effect. It was stable with time and temperature.

### Example 28:W/O/W type Cream

| | | |
|---|---|---|
| 1. | Cetyl isooctanoate | 5.0 |
| 2. | M3T solution 1 obtained in Preparation Example 3 | 6.0 |
| 3. | Octyl methoxy cinnamate | 1.0 |
| 4. | Decamethylcyclopentasiloxane | 5.0 |
| 5. | Methyl glucoside dioleate | 1.5 |
| 6. | Isohexadecane | 3.5 |
| 7. | Magnesium sulfate | 0.5 |
| 8. | Propylene glycol | 5.0 |
| 9. | Purified water | 39.5 |
| 10. | Cetyl alcohol | 1.0 |
| 11. | PEG-10 soya seterol | 2.0 |
| 12. | Antiseptics | q.s. |
| 13. | Perfume | q.s. |
| 14. | Purified water | 30.0 |
| 15. | Cetyl isooctanoate | 5.0 |

### Preparation procedures

A: Components 7 to 9 were mixed.
B: Components 1 and 6 were added to the homogeneous mixture prepared in the step A and emulsified by stirring.
C: Components 10 to 12 and 14 were mixed, to which the emulsion prepared in the step B was added while stirring.
D: To the emulsion obtained in the step C, Component 13 was added.

The cream thus obtained showed good spreadability, non-greasiness, non-tackiness to the touch in addition to UV-protective effect. It lasted long, and was stable with time and temperature.

### Example 29:O/W/O type milky lotion

| Components | | Mass % |
|---|---|---|
| 1. | Glyceryl trioctanoate | 10.0 |
| 2. | Crosslinked polyether-modified silicone ¹⁾ | 5.0 |
| 3. | D5 solution 1 obtained in Preparation Example 1 | 8.0 |
| 4. | Octyl methoxy cinnamate | 1.0 |
| 5. | Sucrose monostearate | 3.0 |
| 6. | Glycerin | 5.0 |
| 7. | 1,3-butylene glycol | 5.0 |
| 8. | Antiseptics | q.s. |
| 9. | Purified water | 59.0 |
| 10. | Macadamia nut oil | 2.0 |
| 11. | Cetyl alcohol | 2.0 |
| 12. | Perfume | q.s. |

| | | |
|---|---|---|
| 1) KSG-210 from Shin-Etsu Chemical Co., Ltd. | | |

### Preparation procedures

A: Components 1 to 4 were mixed.
B: Components 5 and 9 were heat-mixed.
C: Components 10 to 12 were heat-mixed.
D: To the mixture prepared in the step B, the mixture prepared in the step C was added and emulsified by stirring and then cooled.
E: To the mixture prepared in the step A, the emulsion prepared in the step D was added and emulsified.

The milky lotion thus obtained showed good spreadability, non-greasiness, non-tackiness to the touch in addition to UV-protective effect. It lasted long, and was stable with time and temperature.

### Example 30:O/W/O type Liquid foundation

| Components | | Mass % |
|---|---|---|
| 1. | Crosslinked polyether-modified silicone ¹⁾ | 3.0 |
| 2. | Crosslinked dimethylpolysiloxane ²⁾ | 2.0 |
| 3. | Branched alkyl/polyether co-modified silicone³⁾ | 0.5 |
| 4. | D5 solution 2 obtained in Preparation Example 2 | 4.0 |
| 5. | Octyl methoxy cinnamate | 1.0 |
| 6. | Propylene glycol decanoate | 5.0 |
| 7. | Isopropyl myristate | 5.0 |
| 8. | Pigment | 10.0 |
| 9. | Hydrogenated egg yolk derived phospholipid | 1.0 |
| 10. | Glycerin | 2.0 |
| 11. | 1,3-butylene glycol | 10.0 |
| 12. | Antiseptics | q.s. |
| 13. | Purified water | 48.5 |
| 14. | Squalane | 3.0 |
| 15. | Cetyl alcohol | 5.0 |
| 16. | Perfume | q.s. |

| | | |
|---|---|---|
| 1) KSG-210 from Shin-Etsu Chemical Co., Ltd. 2) KSG-15 from Shin-Etsu Chemical Co., Ltd. 2) KF-6038 from Shin-Etsu Chemical Co., Ltd. | | |

### Preparation procedures

A: Components 1 to 7 were mixed.
B: Components 8 and 13 were mixed.
C: Components 14 to 16 were heat-mixed.
D: To the mixture prepared in the step B, the mixture prepared in the step C was added and emulsified by stirring and then cooled.
E: To the mixture prepared in the step A, the emulsion prepared in the step D was added and emulsified.

The liquid foundation thus obtained showed good spreadability, non-greasiness, non-tackiness to the touch in addition to UV-protective effect. It lasted long, and was stable with time and temperature.

### INDUSTRIAL APPLICABILITY

The polymer (A) in the present invention has a UV-absorbing substituent group and an organopolysiloxane branch. It is soluble in decamethylcyclopentasiloxane. Cosmetics comprising the polymer are excellent in UV-protective effect and sensory feel.

## Claims

1. A cosmetic, comprising a polymer (A) which comprises a repeating unit represented by the following formula (1), a repeating unit represented by the following formula (2) and a repeating unit represented by the following formula (3), said polymer (A) containing the repeating unit represented by the formula (3) in an amount of from 30 to 90 mass%, based on a total mass of the polymer (A), and being soluble in decamethylcyclopentasiloxane, 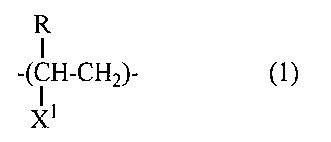 wherein R is a hydrogen atom or a methyl group, X¹ is -COOR¹, wherein R¹ is a hydrogen atom or a C1-10 alkyl group, a phenyl group or an organic group represented by the following formula (4) :
-COO(CₐH₂ₐO)_{b}-R² (4)
wherein R² is a hydrogen atom or a C1-30 monovalent hydrocarbon group, which may have an oxygen atom, a is an integer of from 2 to 5, and b is an integer of from 1 to 100; 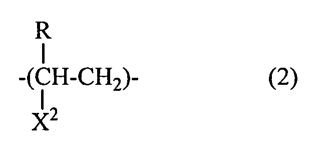 wherein R is as defined above, and X² is an organic group represented by the following formula (5): 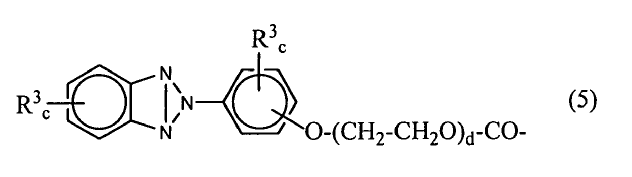 wherein each R³ is, independently, a hydrogen atom, a hydroxyl group, a C1-10 alkyl group or a C1-10 alkoxy group, c is an integer of from 0 to 4, and d is an integer of from 0 to 5; 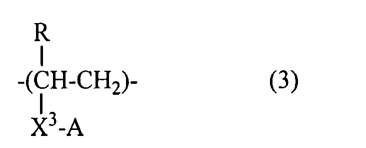 wherein R is as defined above, X³ is a C6-10 divalent aryl group or -C(=O)OR⁴-, wherein R⁴ is a C1-9 alkylene group bonded to A, and A is an organopolysiloxane residue represented by the following formula (6): 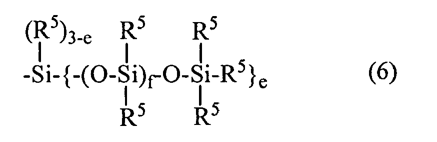 wherein each R⁵ is, independently, a C1-30 alkyl group, aryl group, C1-30 fluorinated alkyl or fluorinated aryl group, e is an integer of from 1 to 3, and f is an integer of from 0 to 300.

2. The cosmetic according to claim 1, wherein X² in the repeating unit represented by the formula (2) is an organic group represented by the following formula (7) or (8).

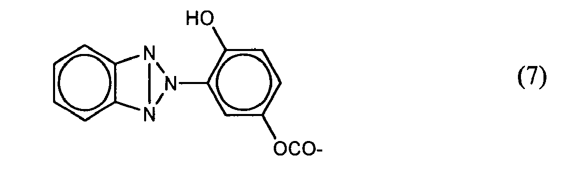

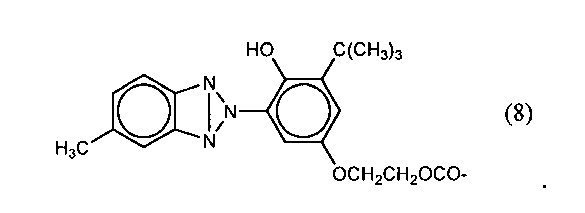

3. The cosmetic according to claim 1 or 2, wherein the polymer (A) contains 0.1 to 70 mass% of the repeating unit represented by the formula (1), and 0.1 to 50 mass% of the repeating unit represented by the formula (2) relative to a total mass of the polymer (A).

4. The cosmetic according to any one of claims 1 to 3, wherein the cosmetic further comprises an ultraviolet light protective agent (B).

5. The cosmetic according to any one of claims 1 to 4, wherein the cosmetic further comprises an unctuous agent (C).

6. The cosmetic according to any one of claims 1 to 5, wherein the cosmetic further comprises water (D).

7. The cosmetic according to any one of claims 1 to 6, wherein the cosmetic further comprises a compound having an alcoholic hydroxyl group (E).

8. The cosmetic according to any one of claims 1 to 7, wherein the cosmetic further comprises a water-soluble or a water-swellable polymer (F).

9. The cosmetic according to any one of claims 1 to 8, wherein the cosmetic further comprises a powder (G).

10. The cosmetic according to claim 9, wherein at least a part of the powder (G) is a spherical fine powder of a crosslinked dimethylpolysiloxane, a spherical fine powder of polymethylsilsesquioxane, or a fine powder of a crosslinked spherical organopolysiloxane rubber coated with polymethylsilsesquioxane particulates.

11. The cosmetic according to any one of claims 1 to 10, wherein the cosmetic further comprises a surfactant (H).

12. The cosmetic according to claim 11, wherein the surfactant (H) is a linear or branched organopolysiloxane having a polyoxyalkylene group, or a linear or branched organopolysiloxane having a polyglycerin moiety, which may further have an alkyl group.

13. The cosmetic according to claim 11 or 12, wherein the surfactant (H) has a HLB of from 2 to 10.

14. The cosmetic according to any one of claims 1 to 13, wherein the cosmetic further comprises a composition consisting of a crosslinked organopolysiloxane having no hydrophilic group and a liquid unctuous agent (I).

15. The cosmetic according to any one of claims 1 to 14, wherein the cosmetic further comprises a composition consisting of a crosslinked organopolysiloxane having a hydrophilic group and a liquid unctuous agent (J).

16. The cosmetic according to claim 15, wherein the crosslinked organopolysiloxane having a hydrophilic group is a crosslinked organopolysiloxane having a polyoxyalkylene group or a crosslinked organopolysiloxane having a polyglycerin moiety.

17. The cosmetic according to any one of claims 1 to 16, wherein the cosmetic further comprises a silicone resin (K).

18. The cosmetic according to claim 17, wherein the silicone resin (K) is an acrylic silicone resin.

19. The cosmetic according to claim 17, wherein the silicone resin (K) is an acrylic silicone resin having at least one group selected from the group consisting of a pyrrolidonyl , a long-chain alkyl, a polyoxyalkylene, a fluoroalkyl, and carboxyl groups.

20. The cosmetic according to claim 17, wherein the silicone resin (K) is at least one selected from the group consisting of silicone resins consisting of R⁶₃SiO_{0.5} and SiO₂ units, silicone resins consisting of R⁶₃SiO_{0.5}, R⁶₂SiO and SiO₂ units, silicone resins consisting of R⁶₃SiO_{0.5} and R⁶SiO_{1.5} units, silicone resins consisting of R⁶₃SiO_{0.5}, R⁶₂SiO, and R⁶SiO_{1.5} units, and silicone resins consisting of R⁶₃SiO_{0.5}, R⁶₂SiO, R⁶SiO_{1.5} and SiO₂ units, wherein R⁶ is a hydrogen atom, C1-10 alkyl group or phenyl group.

21. The cosmetic according to claim 20, wherein the silicone resin (K) has at least one group selected from the group consisting of pyrrolidonyl, long-chain alkyl, polyoxyalkylene, fluoroalkyl, and amino groups.

22. The cosmetic according to any one of claims 1 to 21, wherein the cosmetic is a skin care cosmetic, a makeup cosmetic, a hairdressing cosmetic, an antiperspirant cosmetic, or an ultraviolet light protective cosmetic.

23. The cosmetic according to any one of claims 1 to 21, wherein the cosmetic is in the form of liquid, milky lotion, cream, solid, paste, gel, powder, pressed powder, mousse, spray, or stick.

24. The cosmetic according to any one of claims 1 to 22, wherein the cosmetic is in the form of aqueous, oily, oil-in-water emulsion, water-in-oil emulsion, non-aqueous emulsion, water-in-oil-in-water emulsion or oil-in-water-in-oil emulsion.
